# DEMANDE DE BREVET EUROPEEN

(11) **EP 2 193 784 A1**
(43) Date de publication de la demande: **09.06.2010**
(21) Numéro de dépôt: 09175172.7
(22) Date de dépôt: 05.11.2009
(51) Int. Cl.: A61K 8/49, A61Q 17/04, A61K 8/58

(54) **Compositions cosmetiques comprenant un derive ester de 2-pyrrolidinone 4- carboxy et un filtre lipophile triazine ; utilisation dudit derive comme solvant d'un filtre lipophile triazine**

(30) Priorité: 08.12.2008 FR 0858339
(71) Demandeur: L'Oréal, 75008 Paris (FR)
(72) Inventeur: Richard, Hervé, 93320, Gagny (FR); Muller, Benoit, 75010, Paris (FR)
(74) Mandataire: Miszputen, Laurent

(57) **Abrégé**

La présente invention porte sur une composition comprenant dans un milieu cosmétiquement acceptable au moins un dérivé ester de 2-pyrrolidinone 4-carboxy de formule (1) suivante : 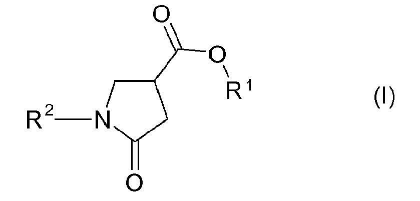 dans laquelle :
R¹ désigne un radical alkyle en C₁-C₂₀, linéaire ou ramifié.
R² désigne un radical alkyle en C₁-C₂₀, linéaire ou ramifié pouvant contenir un cycle en C₅-C₆, le radical phényle, le radical benzyle ou le radical phénéthyle.

et au moins un filtre UV lipophile du type triazine.

La présente invention concerne également l'utilisation d'au moins un dérivé ester de 2-pyrrolidinone 4-carboxy de formule (1) tel que défini ci-dessus, dans une composition comprenant dans un milieu cosmétiquement acceptable au moins un filtre UV lipophile du type triazine, comme solvant dudit filtre UV triazine dans et/ou comme agent améliorant la solubilité dudit actif dans ladite composition ou dans le but d'améliorer le facteur de protection solaire.

## Description

La présente invention concerne une composition comprenant dans un milieu cosmétiquement acceptable au moins un dérivé ester de 2-pyrrolidinone 4-carboxy de formule (1) dont on donnera la définition ci-après et au moins un filtre UV lipophile triazine.

La présente invention est relative à l'utilisation d'au moins un dérivé ester de 2-pyrrolidinone 4-carboxy de formule (1) dont on donnera la définition ci-après, dans une composition comprenant ; dans un milieu cosmétiquement acceptable, au moins un filtre UV lipophile triazine, comme solvant dudit filtre triazine et/ou comme agent améliorant la solubilité dudit filtre UV triazine dans ladite composition.

De nombreux produits cosmétiques ou dermatologiques se présentent sous des formes galéniques diverses comprenant une phase grasse liquide telles que des dispersions, des lotions huileuses, des émulsions huile/eau, eau/huile ou multiple, des gels crèmes. Certains actifs cosmétiques ou dermatologiques particulièrement intéressants comme les filtres organiques lipophiles qui sont difficilement solubles dans la phase huileuse de ces formulations et qui ont tendance durant le stockage à former des cristaux ou à précipiter notamment dans les émulsions. De tels phénomènes sont indésirables d'un point de vue stabilité de la formulation et/ou vis-à-vis du confort du consommateur dans la mesure où ils peuvent déstabiliser la composition et/ou affecter l'apparence esthétique du produit et/ou entraîner des désagréments cosmétiques à l'application sur la peau et/ou les cheveux ou bien à limiter la concentration en actifs dans ces formules ce qui ne permet pas d'obtenir des produits suffisamment efficaces.

En particulier, les compositions antisolaires se présentent souvent sous la forme d'une émulsion de type huile-dans-eau, ou eau-dans-huile, de gels ou de produits anhydres qui contiennent, à des concentrations diverses, un ou plusieurs filtres organiques et/ou inorganiques, insolubles et/ou solubles, lipophiles et/ou hydrophiles, capables d'absorber sélectivement le rayonnement UV nocif. Ces filtres et leurs quantités étant sélectionnés en fonction de l'indice de protection recherché. Selon leur caractère lipophile ou au contraire hydrophile, ces filtres peuvent se répartir, respectivement, soit dans la phase grasse, soit dans la phase aqueuse, de la composition finale.

Les filtres UV du type triazine sont des filtres lipophiles UVB particulièrement intéressants pour les formulations cosmétiques solaires. Certains d'entre eux peuvent filtrer les radiations UVA. Cependant leur pouvoir photoprotecteur en formulation est assez limité dans les supports cosmétiques habituels contenant des huiles telles que les (mono/poly)alcools gras oxyéthylénés ou oxypropylénés ("CETIOL HE" de chez Henkel ou "WITCONOL APM" de chez Witco) ou bien les esters gras tels que le benzoate d'alcools en C12-C15 ("FINSOLV TN" de chez Finetex), les triglycérides d'acides gras, par exemple le Miglyol® 812 commercialisé par la société DYNAMIT NOBEL, les dérivés d'acide aminés ("Eldew SL205" de chez Ajinomoto), à cause d'une solubilité de ces filtres dans ces huiles couramment utilisées en formulation pas complètement satisfaisante. Ceci a pour conséquence : soit l'apparition au cours du temps, de cristallisation dans les formules qui nuisent à la bonne qualité, stabilité et efficacité des produits solaires; soit à limiter la concentration en filtres dans les formules ce qui ne permet pas d'obtenir des produits suffisamment efficaces.

Il existe donc le besoin de trouver de nouveaux solvants permettant de dissoudre efficacement les filtres lipophiles du type triazine pour améliorer leur solubilité dans les huiles dans les supports de formulations cosmétiques ou dermatologiques les contenant sans les inconvénients énumérés ci-dessus.

On sait que les dérivés de pyrrolidone sont connus comme agent de pénétration d'actifs comme l'oléocanthal dans la demande W02008/01240 ou comme les esters d'alkyl d'asprotadil dans le brevet US6,673,841.

Or, la Demanderesse vient maintenant de découvrir, de façon surprenante, une nouvelle famille de solvants efficaces constituée par des dérivés esters de 2-pyrrolidinone 4-carboxy de formule (1) dont on donnera la définition ci-après, permettant d'atteindre cet objectif. Ces composés peuvent être incorporés dans de nombreux produits cosmétiques.

Cette découverte est à la base de la présente invention.

La présente invention concerne notamment une composition comprenant dans un milieu cosmétiquement acceptable au moins un dérivé ester de 2-pyrrolidinone 4-carboxy de formule (1) et au moins un filtre UV du type triazine.

La présente invention est relative à l'utilisation d'au moins un dérivé ester de 2-pyrrolidinone 4-carboxy de formule (1) dont on donnera la définition ci-après, dans une composition comprenant dans un milieu cosmétiquement acceptable au moins un filtre UV du type triazine, comme solvant dudit actif et/ou comme agent améliorant la solubilité dudit filtre UV du type triazine dans ladite composition.

La présente invention est relative également à l'utilisation d'au moins un dérivé diester de pyrrolidinone de formule (1) dans une composition comprenant dans un milieu cosmétiquement acceptable au moins un filtre UV lipophile du type triazine dans le but d'améliorer le facteur de protection solaire.

D'autres caractéristiques, aspects et avantages de l'invention apparaîtront à la lecture de la description détaillée qui va suivre.

Par "cosmétiquement acceptable", on entend compatible avec la peau et/ou ses phanères, qui présente une couleur, une odeur et un toucher agréables et qui ne génère pas d'inconforts inacceptables (picotements, tiraillements, rougeurs), susceptibles de détourner la consommatrice d'utiliser cette composition.

Par "filtre lipophile" on entend tout filtre UV organique cosmétique ou dermatologique susceptible d'être complètement dissous à l'état moléculaire dans une phase grasse liquide ou bien d'être solubilisé sous forme colloïdale (par exemple sous forme micellaire) dans une phase grasse liquide.

Les dérivés esters de 2-pyrrolidinone 4-carboxy conformes à l'invention sont choisis par ceux répondant à la formule générale (I) suivante : 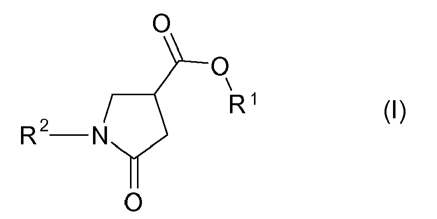 dans laquelle :
R¹ désigne un radical alkyle en C₁-C₂₀, linéaire ou ramifié.
R² désigne un radical alkyle en C₁-C₂₀, linéaire ou ramifié pouvant contenir un cycle en C₅-C₆, le radical phényle, le radical benzyle ou le radical phénéthyle.

Dans la formule (1), parmi les groupes alkyles, on peut notamment citer les groupes méthyle, éthyle, n-propyle, isopropyle, n-butyle, iso-butyle, tert-butyle, n-octyle, éthyle-2 hexyle, dodécyle, héxadécyle, cyclohexyle ou méthyle cyclohexyle.

Parmi les composés de formule (1), on utilisera plus particulièrement les produits (a) à (oo) suivants :

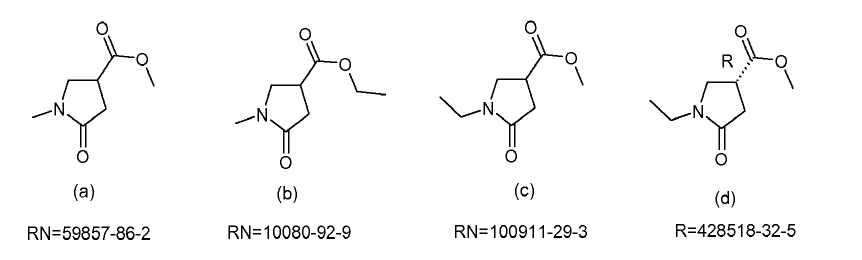

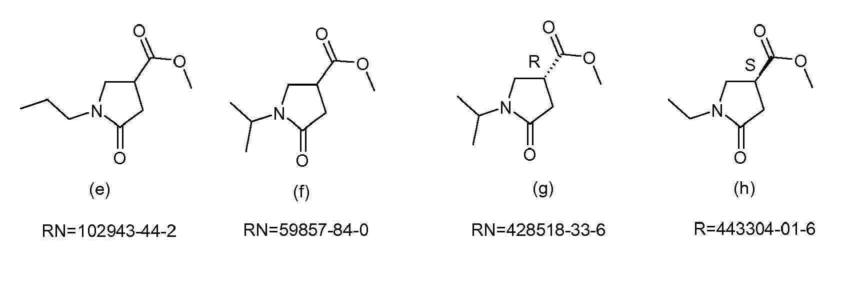

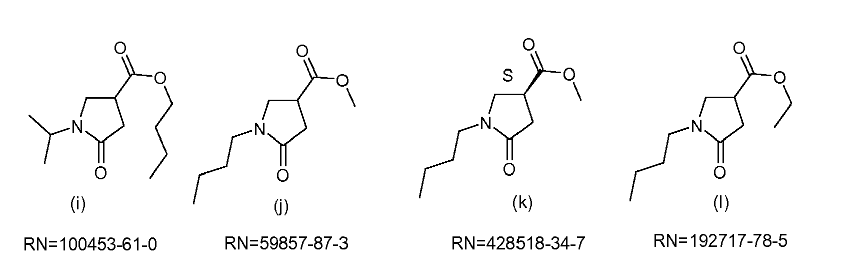

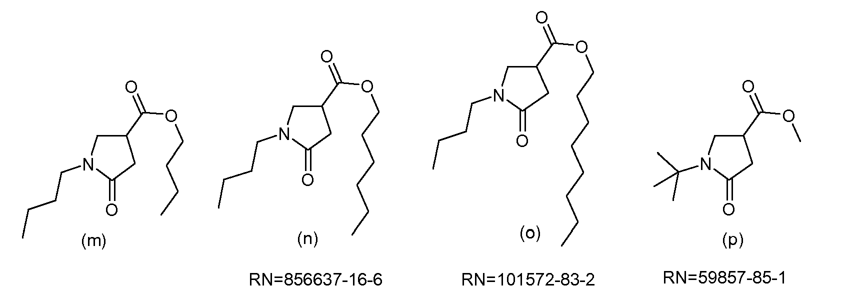

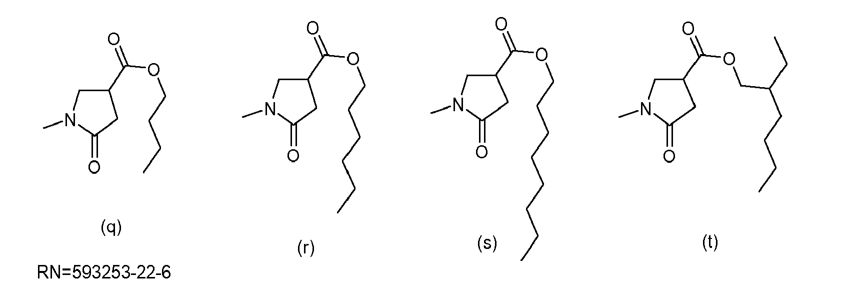

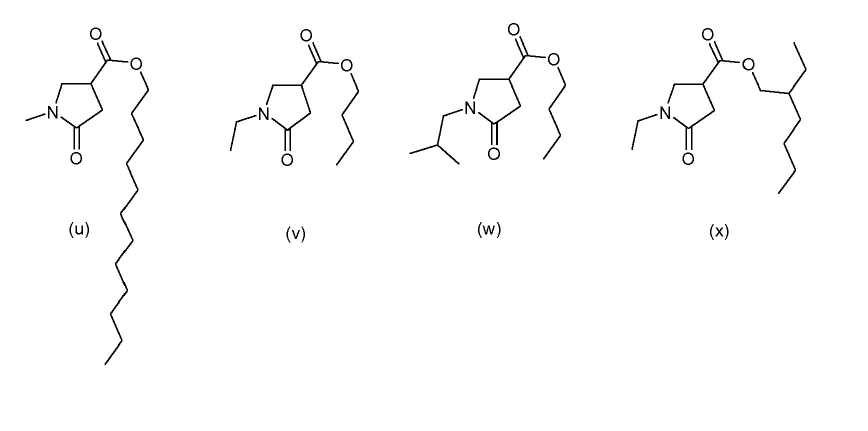

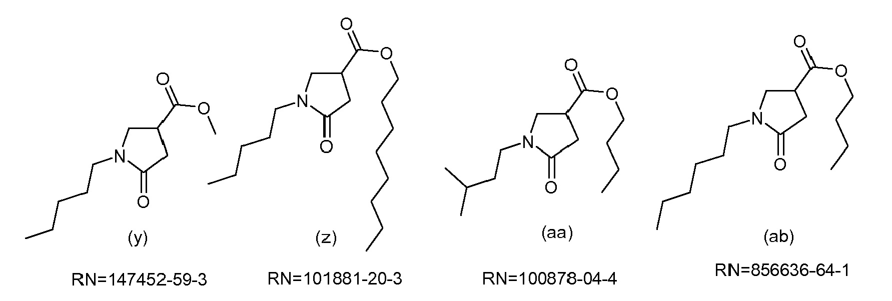

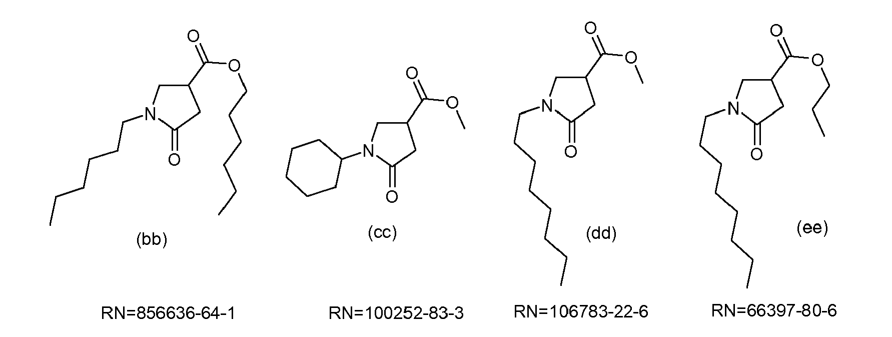

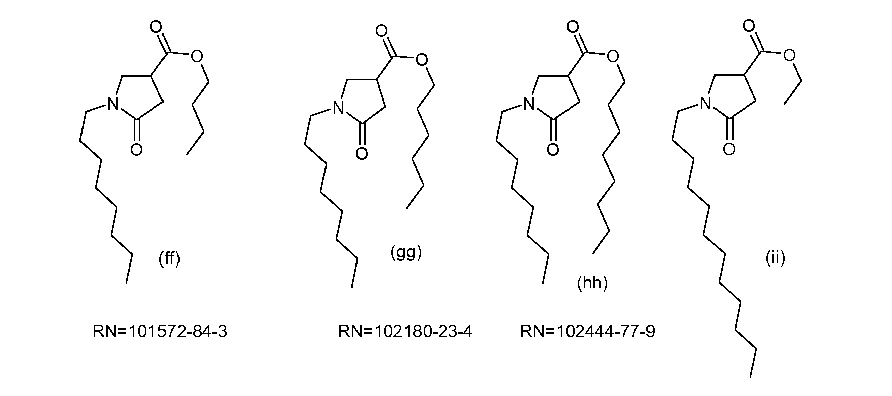

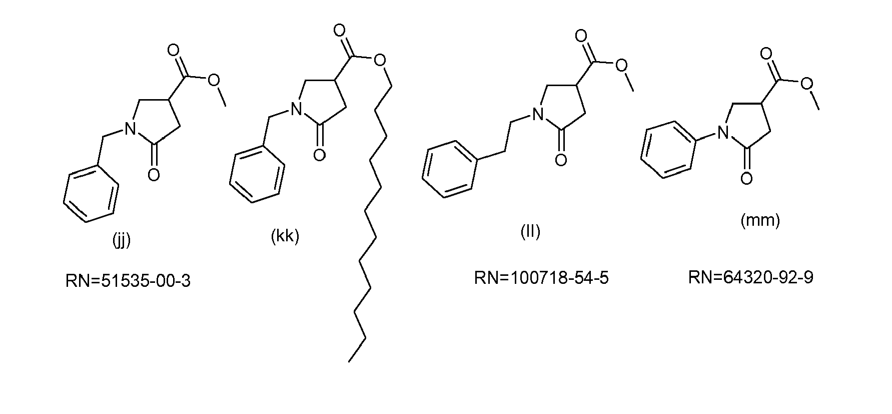

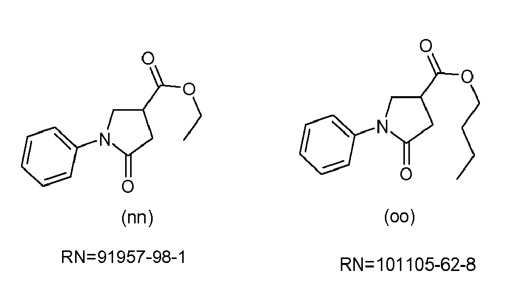

On utilisera plus particulièrement les dérivés des exemples (t), (u), (v), (x), (j), (l), (m), (w), (n), (ab), (ii) et (kk) et encore plus particulièrement les composé (j), (l) et (m) .

Les dérivés de formule (I), dont les synthèses sont décrites dans les articles suivants : J. Org. Chem., 26, pages 1519-24 (1961) ; Tetrahedron Asymmetric, 12 (23), pages 3241-9 (2001) ; J. Industrial & Engineering Chem., 47, pages 1572-8 (1955) ; J. Am. Chem. Soc., 60, pages 402-6 (1938) ; et dans les brevets EP 0069512, US 2811496 (1955), US 2826588, US 3136620, FR 2290199 et FR 2696744 peuvent être facilement obtenus :
- soit par la condensation d'un diester de l'acide itaconique de formule (II) avec une amine primaire de formule (III), avec ou sans solvant à une température comprise entre 20°C et 150°C selon le schéma suivant : 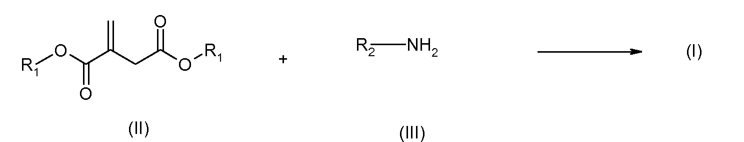
- soit en 2 étapes à partir de l'acide itaconique de formule (IV) par condensation avec l'amine primaire de formule (III) en présence d'un solvant ou pas pour donner l'acide intermédiaire de formule (V), suivi par une estérification de cet acide de formule (V) en présence d'un excès d'alcool de formule (VI) selon le schéma suivant : 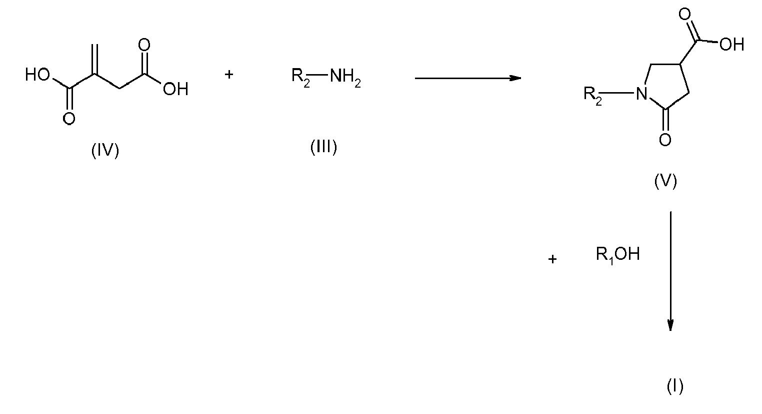
- soit les dérivés de formule (I) à chaîne ester R'₁ (radical alkyle en C₃-C₂₀, linéaire ou ramifié) peuvent également être obtenus par transestérification des dérivés à chaîne ester R₁ (radical méthyle ou éthyle) en présence d'alcool en C₃-C₂₀, linéaire ou ramifié et de catalyseur à l'étain ou au titane selon le schéma suivant :

De façon préférentielle, le ou les dérivés de formule (I) conformes à l'invention sont présents dans les compositions de l'invention à des teneurs allant de 1 à 30% en poids et plus préférentiellement de 3 à 20 % en poids par rapport au poids total de la composition.

Selon une forme particulière de l'invention, le ou les dérivés de formule (I) conformes à l'invention sont présents dans une quantité suffisante permettant de solubiliser à lui-seul ou à eux-seuls (sans qu'il soit nécessaire d'utiliser un autre solvant) la quantité totale de filtre(s) triazine(s) lipophile(s) présent(s) dans la composition.

Les filtres UV du type triazine conformes à l'invention peuvent être notamment choisis parmi les dérivés de 1,3,5-triazine de formule (1) suivante : 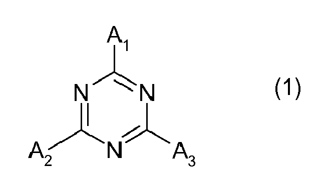 dans laquelle les radicaux A₁, A₂ et A₃, identiques ou différents sont choisis parmi les groupes de formules (2) : 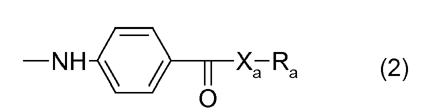 dans laquelle :
- Xₐ, identiques ou différents, représentent l'oxygène ou le radical -NH-;
- Rₐ, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (3), (4) ou (5) suivantes : 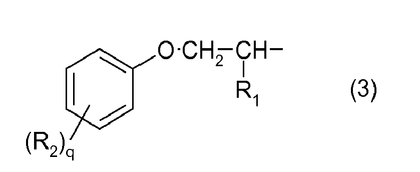 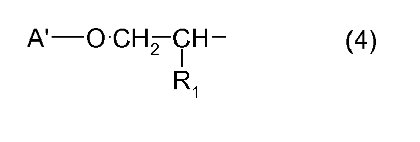 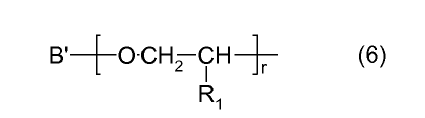 dans lesquelles :
   - R₁ est l'hydrogène ou un radical méthyle;
   - R₂ est un radical alkyle en C₁-C₉;
   - q est un nombre entier allant de 0 à 3;
   - r est un nombre entier allant de 1 à 10;
   - A' est un radical alkyle en C₄-C₈ ou un radical cycloalkyle en C₅-C₈ ;
   - B' est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄.

Une première famille plus particulièrement préférée de dérivés de 1,3,5-triazine, notamment décrite dans le document EP-A-0 517 104, est celle des 1,3,5-triazines répondant à la formule (1) dans laquelle les A₁, A₂ et A₃ sont de formule (2) et présentent les caractéristiques suivantes :
- un des radicaux Xₐ-Rₐ représente le radical -NH-Rₐ avec Rₐ choisi parmi : un radical cycloalkyle en C₅-C₁₂ éventuellement substitué par un ou plusieurs radicaux alkyles en C₁-C₄; un radical de formule (3), (4) ou (5) ci-dessus dans lesquelles :
- B' est un radical alkyle en C₁-C₄ ;
- R₂ est le radical méthyle ;
- les 2 autres Xₐ-Rₐ représentent le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (3), (4) ou (5) ci-dessus dans lesquelles :
- B' est un radical alkyle en C₁-C₄;
- R₂ est le radical méthyle.

Une deuxième famille plus particulièrement préférée de dérivés de 1,3,5-triazine, notamment décrite dans le document EP-A-0 570 838, est celle des 1,3,5-triazines répondant à la formule (1) dans laquelle les A₁, A₂ et A₃ sont de formule (2) et présentant l'ensemble des caractéristiques suivantes :
- un ou deux Xₐ-Rₐ représente le radical -NH-Rₐ , avec Rₐ choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄; un radical de formule (3), (4) ou (5) ci-dessus dans lesquelles :
- B' est un radical alkyle en C₁-C₄ ;
- R₂ est le radical méthyle ;
le ou les deux autres Xₐ-Rₐ étant le radical -O-Rₐ avec Rₐ, identiques ou différents choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (3), (4) ou (5) ci-dessus dans lesquelles :
- B' est un radical alkyle en C₁-C₄;
- R₂ est le radical méthyle.

Une 1,3,5-triazine particulièrement préférée de cette deuxième famille est la 2-[(p-(tertiobutylamido)anilino]-4,6-bis-[(p-(2'-éthylhexyl-1'-oxycarbonyl)anilino]-1,3,5-triazine ou « Diethylhexyl Butamido Triazone » vendue sous le nom commercial « UVASORB HEB » par SIGMA 3V et répondant à la formule suivante : 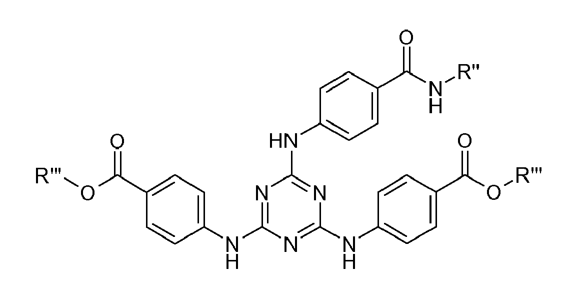 dans laquelle R"' désigne un radical éthyl-2 hexyle et R" désigne un radical tert-butyle.

Une troisième famille préférée de composés utilisables dans le cadre de la présente invention, et qui est notamment décrite dans le document US 4,724,137, est celle des 1,3,5-triazines répondant à la formule (1) dans laquelle les A₁, A₂ et A₃ sont de formule (2) et présentent les caractéristiques suivantes :
- Xₐ sont identiques et représentent l'oxygène;
- Rₐ, identiques ou différents et représentent un radical alkyle en C₆-C₁₂ ou un radical polyoxyéthyléné comprenant de 1 à 6 unités d'oxyde d'éthylène et dont le groupe OH terminal est méthylé.

Une 1,3,5-triazine particulièrement préférée de cette troisième famille est la 2,4,6-tris[p-(2'-éthylhexyl-1'-oxycarbonyl)anilino]-1,3,5-triazine ou « Ethylhexyl Triazone » vendue notamment sous la dénomination commerciale de "UVINUL T 150" par la Société BASF et répond à la formule suivante : 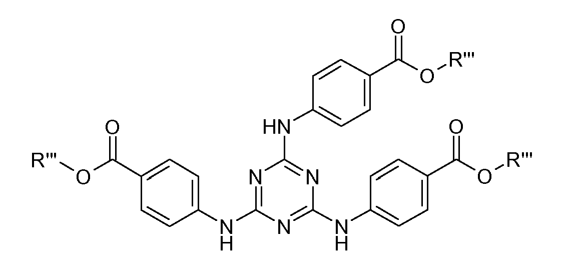 dans laquelle R"' désigne un radical 2-éthyl hexyle.

Les filtres UV du type triazine peuvent être également choisis parmi les dérivés de bis-résorcinyl triazine, conformes à la présente invention répondent à la formule (6) suivante : 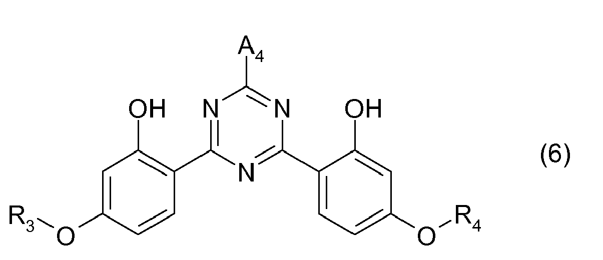 dans laquelle :
les radicaux R₃ et R₄, identiques ou différents, désignent un radical alkyle en C₃-C₁₈; un radical alcényle en C₂-C₁₈ ou bien un reste de formule -CH₂-CH(OH)-CH₂-OT₁ où T₁ est un atome d'hydrogène ou un radical alkyle en C₁-C₈ ;
   - A₄ désigne un reste répondant à l'une des formules suivantes : 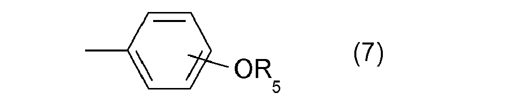 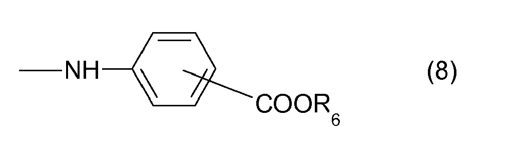 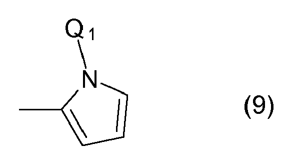
dans lesquelles :
- R₅ désigne un atome d'hydrogène, un radical alkyle en C1-C10, un radical de formule :
   -(CH₂CHR₇-O)ₙ₁R₆ où n₁ est un nombre de 1 à 16 ou bien un reste de structure -CH₂-CH-(OH)-CH₂OT₁ avec T₁ ayant la même signification indiquée ci-dessus ;
- R₆ désigne hydrogène, un cation métallique M, un radical alkyle en C₁-C₅ ou un reste de formule -(CH₂)ₘ₂-OT₁ où m₂ est un nombre de 1 à 4 et T₁ a la même signification indiquée ci-dessus.
- R₇ est hydrogène ou méthyle
- Q₁ est un radical alkyle en C₁-C₁₈.

Dans les formules (6) à (9) décrites ci-dessus :
- les radicaux alkyle sont linéaires ou ramifiés et peuvent être choisis par exemple parmi méthyle, éthyle, n-propyle, isopropyle, n-butyle, sec.butyle, tert.butyle, amyle, isoamyle, tert.amyle, heptyle, octyle, isooctyle, nonyle, décyle, undécyle, dodécyle, tétradécyle, pentadécyle, hexadécyle, heptadécyle ou octadécyle ;
- les radicaux alcényle peuvent être choisis par exemple parmi allyle, méthallyle, isopropènyle, 2-butènyle, 3-butènyle, isobutènyle, n-penta-2,4-diènyle, 3-méthyl-but-2-enyle, n-oct-2-enyle, n-dodec-2-enyle, iso-dodecenyle, n-octadec-4-enyle ;
- les radicaux alcoxy sont linéaires ou ramifiés et peuvent être choisis par exemple parmi méthoxy, éthoxy, n-propoxy, isopropoxy, n-butoxy, sec.-butoxy, tert.-butoxy, amyloxy, isoamyloxy ou tert.amyloxy ;
- les radicaux mono ou dialkylamino en C₁-C₅ peuvent être choisis par exemple parmi méthylamino, éthylamino, propylamino, n-butylamino, sec.butylamino, tert.butylamino, pentylamino, diméthylamino, diéthylamino, dibutylamino ou méthyléthylamino.
- les cations métalliques sont des cations alcalins, alcalino-terreux ou métalliques choisis par exemple parmi lithium, potassium, sodium, calcium, magnésium, cuivre et zinc.

Les dérivés de bis-résorcinyl triazine de formule (6) de l'invention sont des filtres déjà connus en soi. Ils sont décrits et préparés selon les synthèses indiquées dans les demandes de brevet EP-A-0775 698.

A titre d'exemples de composés de formule (6) utilisables, on peut citer :
- la 2,4-bis {[4-(2-éthyl-hexyloxy)-2-hydroxy]-phényl}-6-(4-méthoxy-phenyl)-1,3,5-triazine ;
- la 2,4-bis{[4-(3-(2-propyloxy)-2-hydroxy-propyloxy)-2-hydroxy]-phényl}-6-(4-méthoxyphényl)-1,3,5-triazine ;
- la 2,4-bis {[4-(2-éthyl-hexyloxy)-2-hydroxy]-phényl}-6-[4-(2-méthoxyéthyl-carboxyl)-phénylamino]-1,3,5-triazine ; 1,3,5-triazine ;
- la 2,4-bis {[4-(2"-méthylpropenyloxy)-2-hydroxy]-phényl}-6-(4-méthoxyphényl)-1,3,5-triazine ;
- la 2,4-bis{[4-(3-(2-propyloxy)-2-hydroxy-propyloxy]-2-hydroxy]-phényl}-6-[(4-éthylcarboxyl)-phénylamino]-1,3,5-triazine ;
- la 2,4-bis {[4-(2-éthyl-hexyloxy)-2-hydroxy]-phényl}-6-(1-méthylpyrrol-2-yl)-1,3,5-triazine.

Le composé dérivé de bis-résorcinyl triazine plus particulièrement préféré selon l'invention sera le composé 2,4-bis {[4-(2-éthyl-hexyloxy)-2-hydroxy]-phényl}-6-(4-méthoxy-phényl)-1,3,5-triazine (nom INCI : Bis-Ethylhexyloxyphenol Methoxyphenyl Triazine » vendu sous le nom commercial «TINOSORB S » par CIBA GEIGY,

On peut également citer les esters de benzalmalonate sur s-triazine tels que décrits dans les demandes de brevet EP507691, EP507692, EP790243 et EP944624 et en particulier les composés suivants :
2,4,6-tris-(4'-amino benzalmalonate de diisobutyle)-6-butoxy s-triazine,
2,4,6 tris-(4'-amino benzalmalonate de diisobutyle)-6-(2-ethylhexylamino)-s-triazine
2,4-bis-(4'-aminobenzylidène camphre)-6-(2-ethylhexyl-amino)-triazine,
2,4,6 -bis-(4'aminobenzylidène camphre)-6-(4'-aminobenzalmalonate de diisobutyle)-s-triazine,
2,4 6-tris(4'-amino benzalmalonate de diéthyle)-s-triazine
2,4,6-tris-(4'-amino benzalmalonate de diisopropyl)-s-triazine,
et plus particulièrement le 2,4,6-tris-(4'-amino benzalmalonate de diisobutyle)-s-triazine

Les filtres UV du type triazine peuvent être également choisis parmi décrits dans les brevets EP 0841341, EP 1471059, EP 1568700 et EP 1642893, EP 1815884.

On peut citer par exemple
- la 2,4,6-tris(4'-amino benzalmalonate de dinéopentyle)-s-triazine
- la 2,4bis (4'-aminobenzoate de n-butyle)-6-(aminopropyltrisiloxane)-s-triazine,

Les filtres UV du type triazine peuvent être en particulier choisis parmi ceux répondant à la formule générale (9) suivante ou l'une de ses formes tautomères 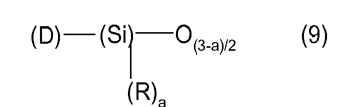 dans laquelle
- R, identiques ou différents représentent un radical alkyle en C₁-C₃₀, linéaire ou ramifié et éventuellement halogéné ou insaturé, un radical aryle en C₆-C₁₂, un radical alkoxy en C₁-C₁₀, un radical hydroxy ou le groupe triméthylsilyloxy ;
- a = 1 à 3 ; en plus des unités de formule -A-(Si)(R)ₐ(O)_{(3-a)/2},

L'organosiloxane peut comporter des unités de formule : (R)_{b}-(Si)(O)_{(4-b)/2} dans lesquelles R a la même signification que dans la formule (9), b = 1, 2 ou 3 ;
- le groupe (D) désigne un composé s-triazine de formule (10) suivante : 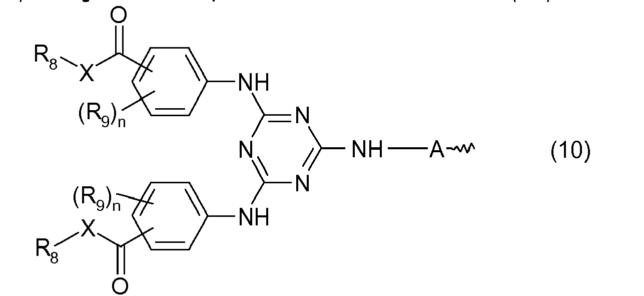 où
   - X représente -O- ou -NR₁₀-, avec R₁₀ qui représente l'hydrogène ou un radical alkyle en C₁-C₅,
   - R₈ représente un radical alkyle en C₁-C₃₀, linéaire ou ramifié et éventuellement insaturé et pouvant contenir un atome de silicium, un groupe cycloalkyle en C₅-C₂₀, éventuellement substitué par 1 à 3 radicaux alkyles en C₁-C₄, linéaires ou ramifiés, le groupe -(CH₂CHR₁₀-O)ₘR₁₁ ou le groupe -CH₂-CH(OH)-CH₂-O-R₁₂,
   - R₉, identiques ou différents, représentent un radical hydroxy, un radical alkyle en C₁-C₈, linéaire ou ramifié, un radical alcoxy en C₁-C₈, deux R₂ adjacents d'un même noyau aromatique pouvant former ensemble un groupement alkylidène dioxy dans lequel le groupe alkylidène contient 1 ou 2 atomes de carbone,
   - R₁₀ représente l'hydrogène ou méthyle ; le groupement (C=O)XR₈ pouvant être en position ortho, méta ou para du groupement amino,
   - R₁₁ représente l'hydrogène ou un groupe alkyle en C₁-C₈,
   - R₁₂ représentent l'hydrogène ou un groupe alkyle en C₄-C₈,
   - m est un nombre entier allant de 2 à 20,
   - n = 0 à 2,
   - A est un radical divalent choisi parmi méthylène ou un groupe répondant à l'une des formules (11), (12) , (13) ou (14) suivantes : 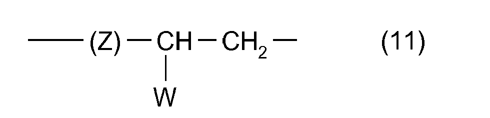 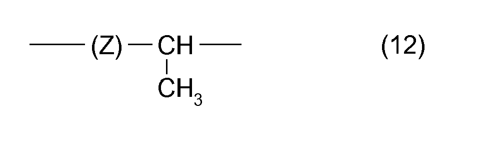 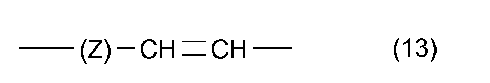 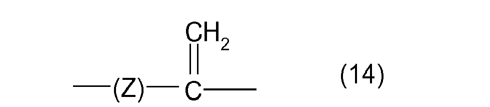 dans lesquelles :
      - Z est un diradical alkylène en C₁-C₁₀, linéaire ou ramifié, saturé ou insaturé, éventuellement substitué par un radical hydroxyle ou des oxygènes et pouvant éventuellement contenir un groupement amino,
      - W représente un atome d'hydrogène, un radical hydroxyle ou un radical alkyle en C₁-C₈, linéaire ou ramifié, saturé ou insaturé,

Il est à noter que les dérivés de formule (9) peuvent être utilisés sous leurs formes tautomères et plus particulièrement sous la forme tautomère de formule (9') suivante : 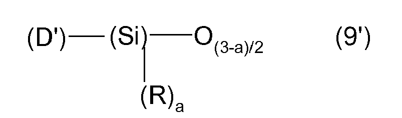 dans laquelle le groupe (D') désigne un composé s-triazine de formule (10') suivante :

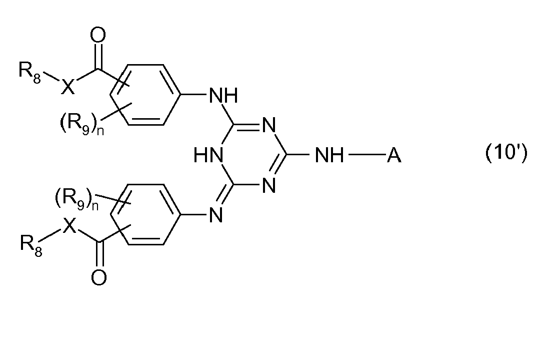

En plus des unités de formule-A-(Si)(R)ₐ(O)_{(3-a)/2}, l'organosiloxane peut comporter des unités de formule (R)_{b}-(Si)(O)_{(4-b)/2} dans lesquelles R a la même signification que dans la formule (9), b = 1, 2 ou 3.

Dans les formules (9) et (9') telles que définies ci-dessus, les radicaux alkyles peuvent être linéaires ou ramifiés, saturés ou insaturés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et tert-octyle. Le radical alkyle particulièrement préféré est le radical méthyle.

Les dérivés de s-triazine préférentiels sont ceux pour lesquels dans la formule (9) ou (9') au moins l'une, et encore plus préférentiellement l'ensemble, des caractéristiques suivantes sont remplies :
R est méthyle,
a = 1 ou 2,
R₈ est un radical en C₂-C₈,
Z = -CH₂- ,
W = H.

De manière préférée, les composés s-triazine de formule (9) de l'invention sont représentés par les formules (9a), (9b) ou (9c) suivantes :

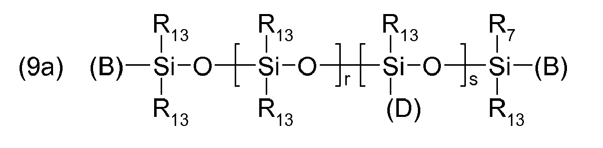

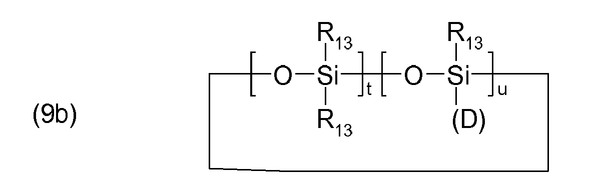

(9c) (D)-Si(R₁₄)₃

dans lesquelles :
- (D) répond à la formule (10) telle que définie ci-dessus,
- R₁₃, identiques ou différents, sont choisis parmi les radicaux alkyles linéaires ou ramifiés en C₁-C₂₀, phényle, 3,3,3-trifluoropropyle et triméthylsilyloxy ou le radical hydroxy,
- R₁₄, identiques ou différents, sont choisis parmi les radicaux alkyles et alcényles, linéaires ou ramifiés en C₁-C₂₀ , les radicaux hydroxy ou phényle,
- (B), identiques ou différents sont choisis parmi les radicaux R₁₃ et le radical (D),
- r est un nombre entier compris entre 0 et 200 inclusivement,
- s est un nombre entier allant de 0 à 50 et si s = 0, au moins l'un des deux symboles (B) désigne (D),
- u est un nombre entier allant de 1 à 10,
- t est un nombre entier allant de 0 à 10, étant entendu que t + u est égal ou supérieur à 3 ainsi que leurs formes tautomères.

Les diorganosiloxanes linéaires de formule (9a) sont particulièrement préférés.

Les diorganosiloxanes linéaires ou cycliques de formule (9a) ou (9b) rentrant dans le cadre de la présente invention, sont des oligomères ou polymères statistiques présentant de préférence au moins l'une, et encore plus préférentiellement l'ensemble, des caractéristiques suivantes :
- R₁₃ est le radical méthyle, le radical alcoxy en C₁-C₂ ou le radical hydroxy,
- B est préférentiellement méthyle (cas des composés linéaires de formule (9a)),

A titre d'exemples de composés de formule (9) particulièrement préférés, on citera les composés de formules (9₁) à (9₁₄) suivantes ainsi que leurs formes tautomères : 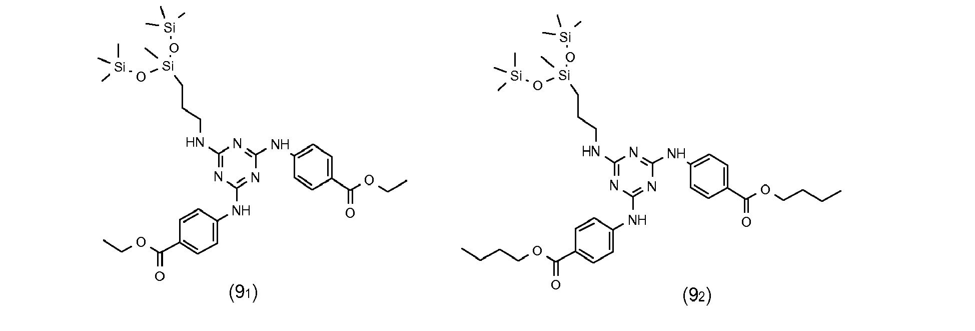 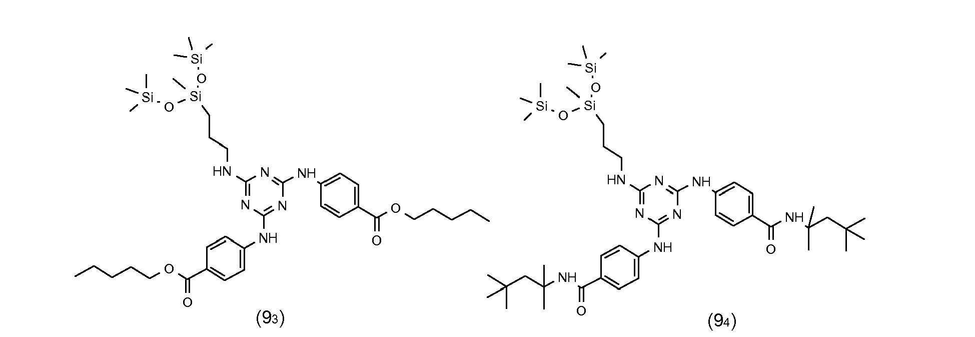 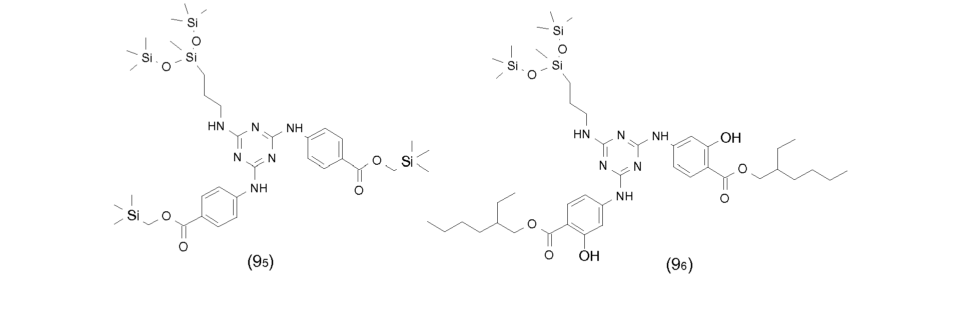 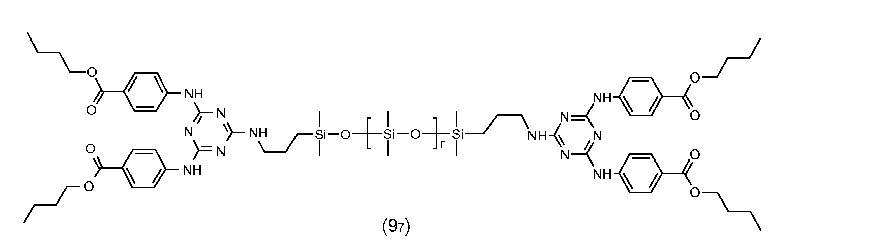 avec r = 8,1

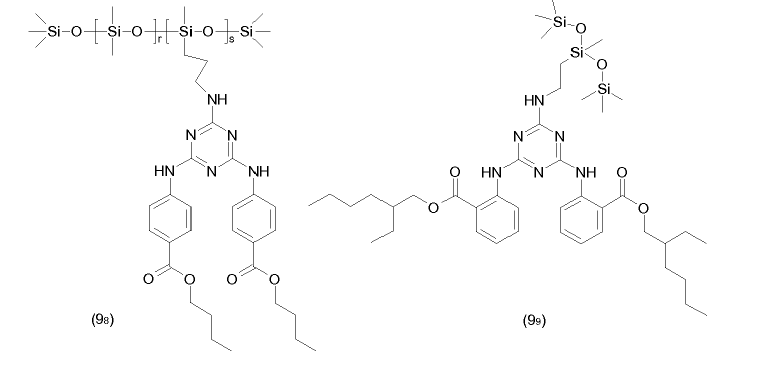

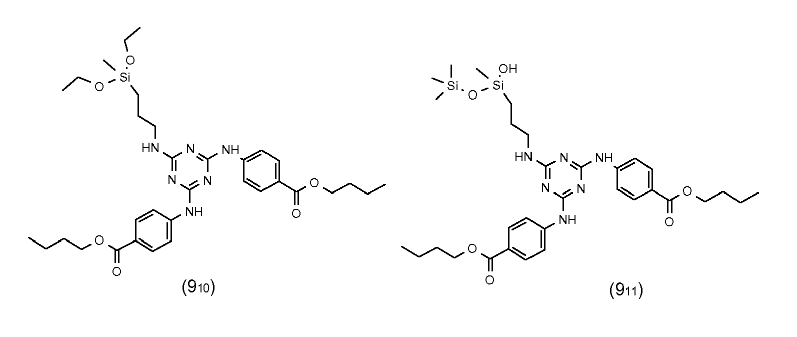

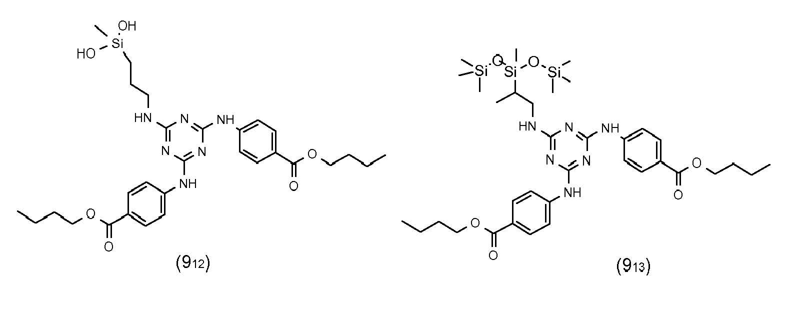

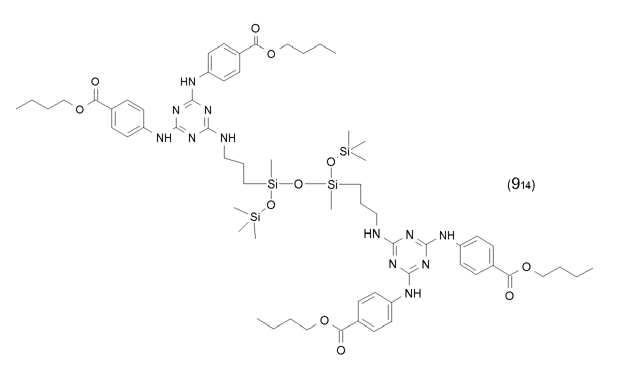

On utilisera plus particulièrement le composé 2,4-bis(4'-diylamino benzoate de n-butyle)-6-{[1,3,3,3-tétraméthyl-1-[(triméthylsilyl)oxy]disiloxanyl]propyl-3-ylamino}-s-triazine de structure (9₂) :

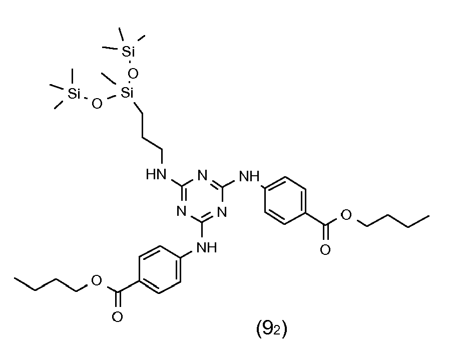

Les triazines de formule (9) ont été décrits ainsi que leur synthèse dans la demande de brevet EP1891079.

Les triazines lipophiles conformes à la présente invention peuvent être choisis parmi ceux répondant à la formule générale (15) suivante : 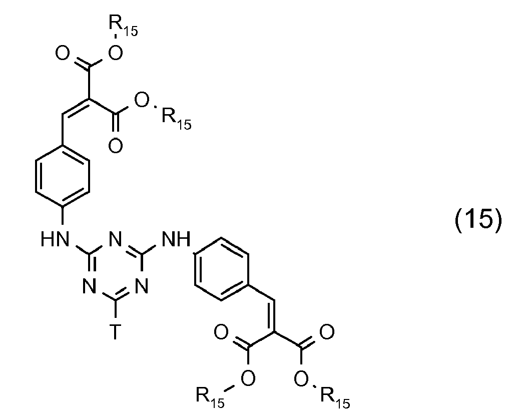 dans laquelle :
R₁₅, identiques ou différents, désignent un groupe de formule (16) : 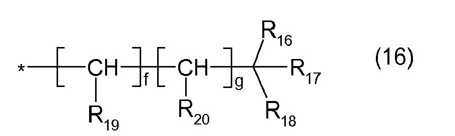 dans laquelle :
   R₁₆ et R₁₇, identiques ou différents représentent un groupe alkyle en C₁-C₈,
   linéaire ou ramifié,
   R₁₆ et R₁₇ peuvent former un cycle en C₅-C₈, éventuellement substitué par 1, 2 ou 3 groupements alkyle(s) en C₁-C₄, linéaire(s) ou ramifié(s) ;
   R₁₈, R₁₉ et R₂₀, identiques ou différents représentent un atome d'hydrogène ou
   un groupe alkyle en C₁-C₄, linéaire ou ramifié;
   f vaut 0 ou 1 ;
   g vaut 0 ou 1 ;
   sous réserve que :
      - lorsque f = 1 et R₁₉ désigne l'hydrogène alors g est égal à 0 et R₁₈ est différent de l'hydrogène,
      - lorsque R₁₆ et R₁₇ forment un cycle en C₅-C₈ alors la somme f+g est différente de 2;
   T est un radical divalent :
      - de formule (17) suivante : 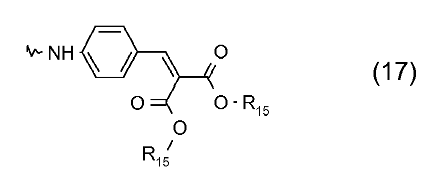 dans laquelle R₁₅ a la même définition que dans la formule (15) ci-dessus ;
      - de formule (18) suivante : 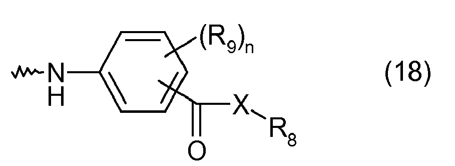 dans laquelle R₈, R₉, X et n ont la même définition que dans la formule (9) ci-dessus ;
      - de formule (19) suivante : 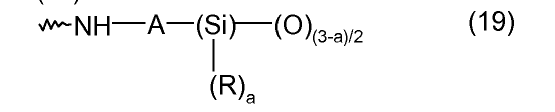
      - ou de formule (20) suivante : 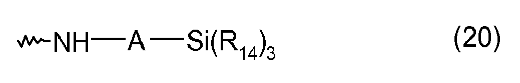
   dans laquelle A et R₁₄ ont les mêmes définitions que dans la formule (9c) ci-dessus.

En plus des unités de formule (R)a-(Si)(O)_{(4-a)/2}, l'organosiloxane peut comporter des unités de formule : (R)_{b}-(Si)(O)_{(4-b)/2} dans lesquelles R, a, A ont les mêmes définitions qu'à la formule (I) ci-dessus et b vaut 1,2 ou 3.

Parmi les composés de formule (15) préférés, on citera ceux pour lesquels :
- f=1 et R₁₉ désigne un alkyle en C₁-C₄ et plus particulièrement méthyle,
- f=1 et R₁₈ désigne un alkyle en C₁-C₄ et plus particulièrement méthyle,
- f=g=0 et R₁₆, R₁₇, R₁₈ désignent un alkyle en C₁-C₄ et plus particulièrement méthyle,
- ou bien R₁₈ désigne hydrogène et R₁₆ et R₁₇ forment un cycle en C₅-C₈ éventuellement substitué par 1 ou 2 radicaux alkyle et plus particulièrement cyclohexyle.

Parmi les composés de formules (15) où T désigne un groupe de formule (16) particulièrement préférés, on citera ceux choisis parmi les composés de formules (15₁) à (15₅) suivantes :

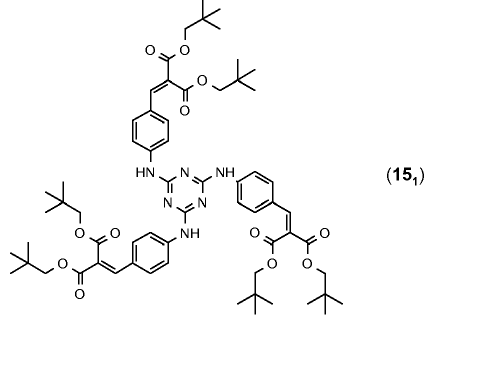

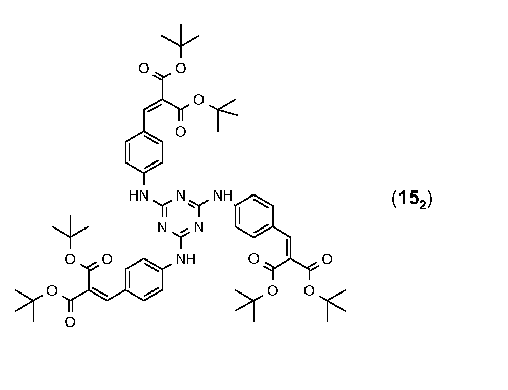

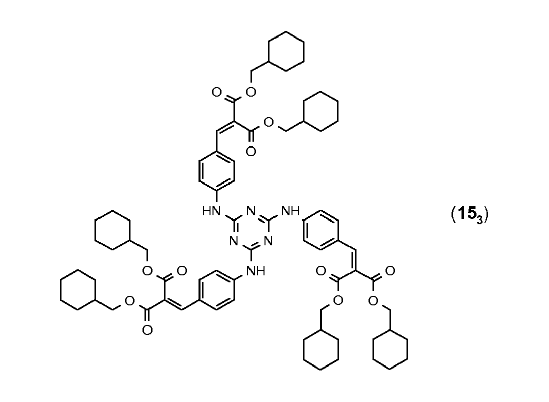

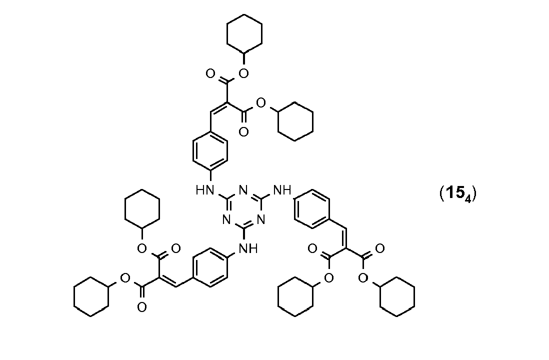

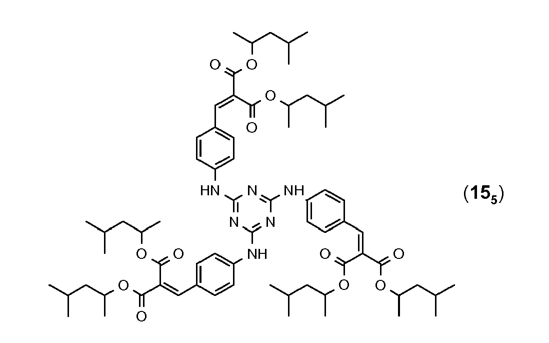

Parmi les composés de formules (IX) où T désigne un groupe de formule (17) particulièrement préférés, on citera ceux choisis parmi les composés de formules (15₆₎ à (15₁₄) suivantes :

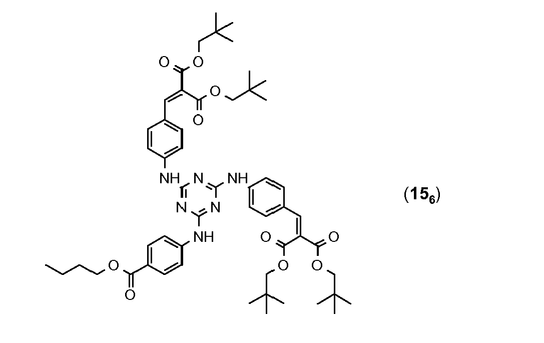

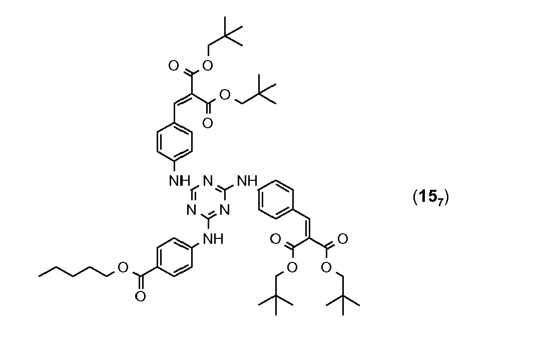

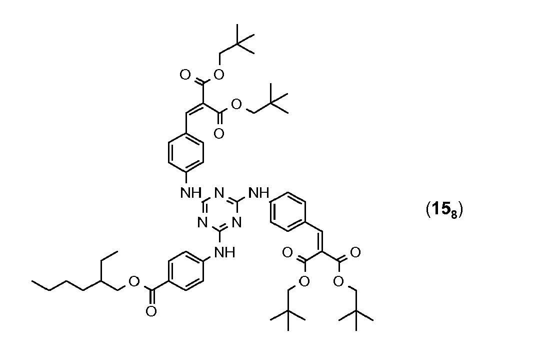

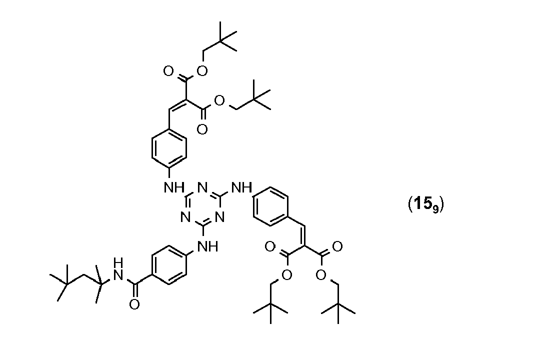

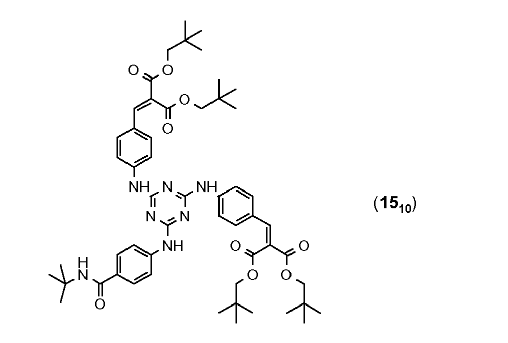

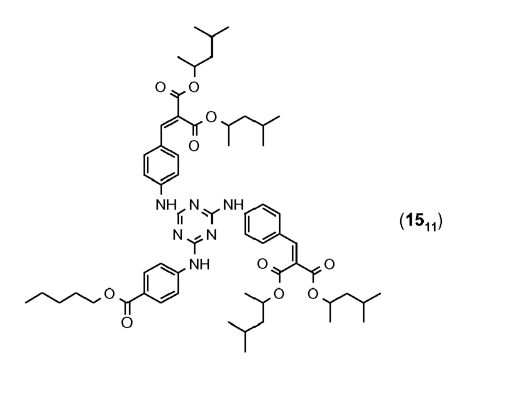

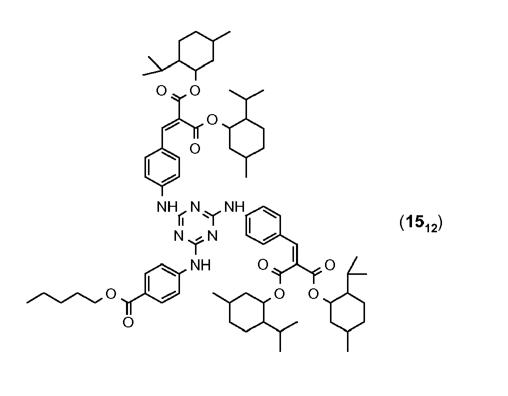

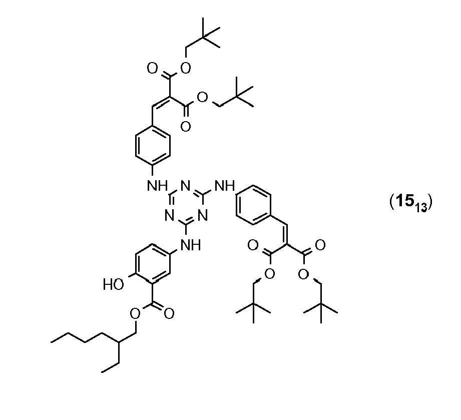

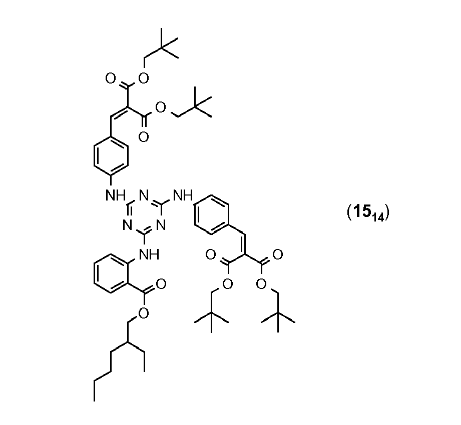

Parmi les composés de formules (IX) où T désigne un groupe de formule (18) particulièrement préférés, on citera ceux choisis parmi les composés de formules (15₁₅) et (15₁₉) suivantes :

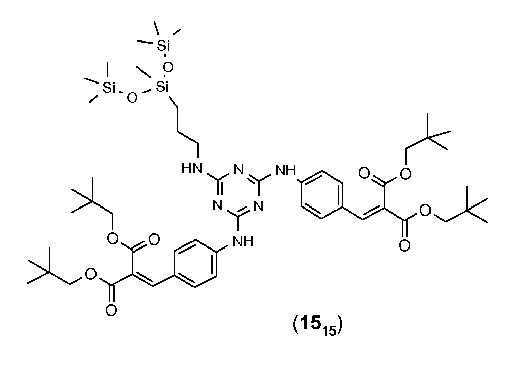

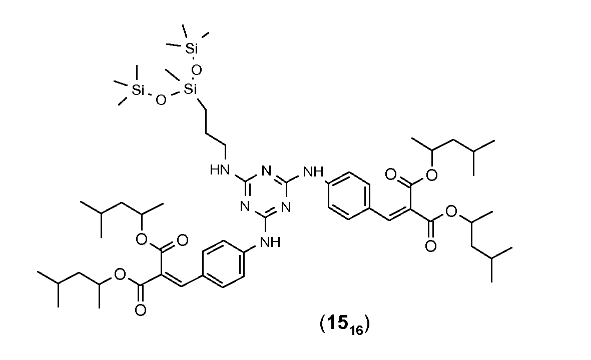

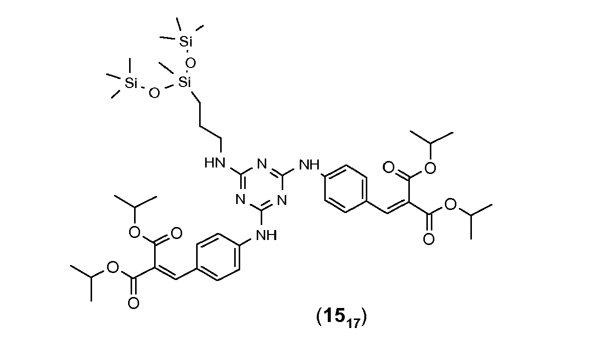

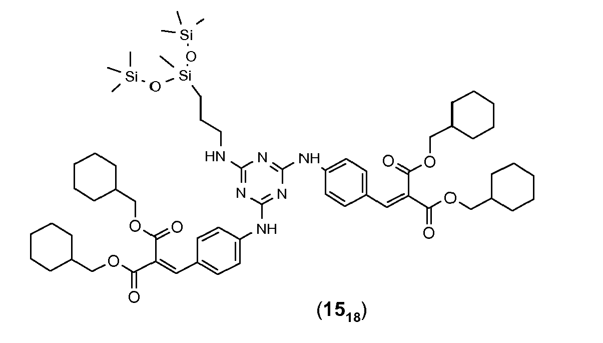

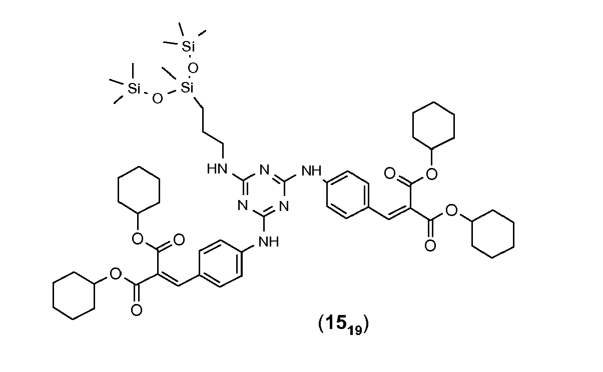

Parmi les composés de formules (VII) où T désigne un groupe de formule (19) particulièrement préférés, on citera ceux choisis parmi les composés de formules (15₂₀₎ et (15₂₁) suivantes :

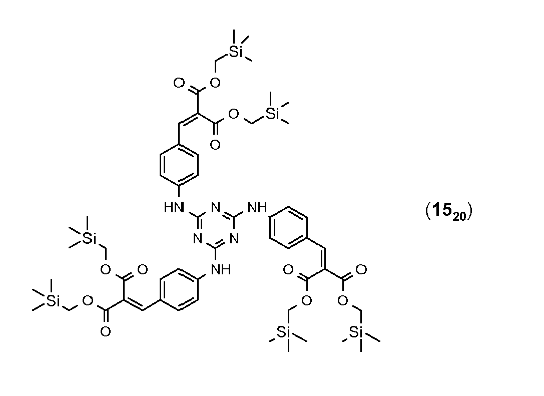

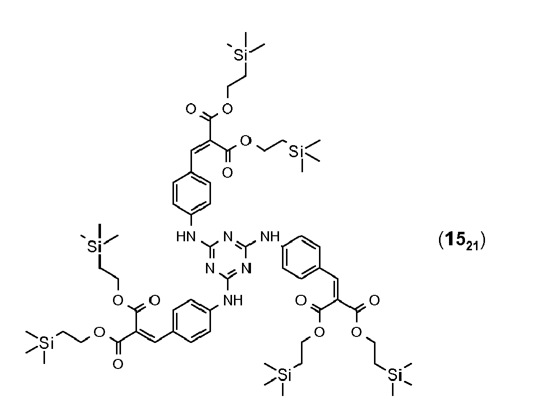

Parmi ces composés de formule (15), on utilisera plus particulièrement les composés **(15₁) , (15₅) , (15₆) , (15₁₀) , (15₁₅) , (15₁₇)** et **(15₂₀).**

Parmi ces composés, on utilisera plus particulièrement et le dineopentyl 2-[4-({4,6-bis[(4-{3-(neopentyloxy)-2-[(neopentyloxy)carbonyl]-3-oxoprop-1-enyl}phenyl)amino]-1,3,5-triazin-2-yl}amino)benzylidene]malonate de formule **(15₁)** : 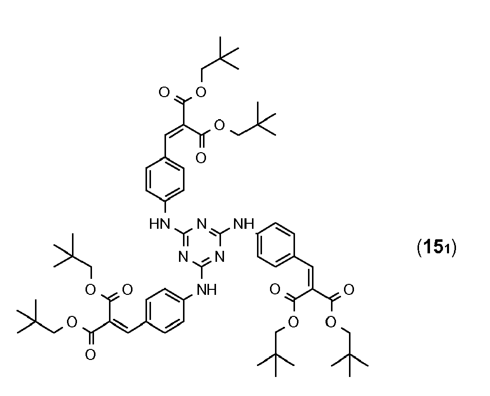 et la 2,4-bis-(4'-amino benzalmalonate de di-néopentyle)-6-(4"-amino benzoate de butyle)-s-triazine de formule (**15₆**) :

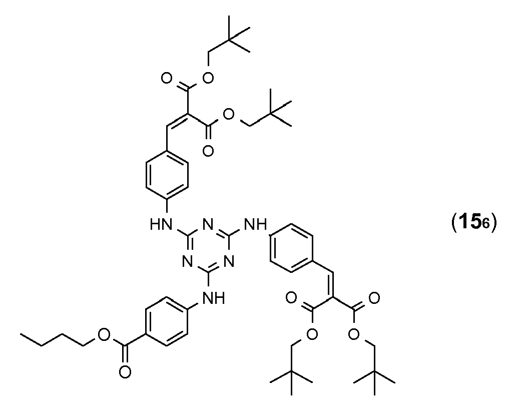

Comme autres exemples de filtres lipophiles du type triazine on peut citer également les dérivés de benzoxazole tels que décrits dans les demandes de brevet EP0832642, EP1027883, EP1300137 et DE10162844 comme le 2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V.

Selon une forme particulière de l'invention, le ou les dérivés de formule (I) conformes à l'invention sont présents dans une quantité suffisante permettant de solubiliser à lui-seul ou à eux-seuls (sans qu'il soit nécessaire d'utiliser un autre solvant) la quantité totale d'actif(s) lipophile (s) présent(s) dans la composition.

Les filtres UV triazine conformes à l'invention sont de préférence présents dans les compositions conformes à l'invention à des teneurs de 0,01 à 20% en poids et plus préférentiellement de 0,1 à 10 % et encore plus préférentiellement de 0,1 à 6% en poids par rapport au poids total de la composition.

Les compositions de l'invention peuvent contenir en plus d'autres filtres UV complémentaires actifs dans l'UVA et/ou l'UVB.

Les filtres organiques complémentaires sont notamment choisis parmi les anthranilates ; les dérivés de dibenzoylméthane ; les dérivés cinnamiques ; les dérivés salicyliques, les dérivés du camphre ; les dérivés de la benzophénone ; les dérivés de β,β-diphénylacrylate , les dérivés de triazine hydrosolubles ou insolubles dans les huiles et dans l'eau ; les dérivés de benzotriazole ; les dérivés de benzalmalonate notamment ceux cités dans le brevet US5624663 ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 ; les mérocyanines tels que décrits dans les demandes W02004006878, W02006/003094, le document IP COM Journal 4 (4), 16 N°IPCOM000011179D publié le 04/03/2004, les demandes de brevet EP1965870, EP1962786 , EP1965869 et leurs mélanges.

Comme exemples d'agents photoprotecteurs organiques complémentaires, on peut citer ceux désignés ci-dessous sous leur nom INCI :

### Dérivés du dibenzoylméthane :

Butyl Methoxydibenzoylmethane vendu notamment sous le nom commercial « PARSOL 1789 » par HOFFMANN LAROCHE,
Isopropyl Dibenzoylmethane,

### Dérivés cinnamiques :

Ethylhexyl Methoxycinnamate vendu notamment sous le nom commercial PARSOL MCX par DSM Nutritional Products, Inc,,
Isopropyl Methoxycinnamate

Isoamyl Methoxycinnamate vendu sous le nom commercial NEO HELIOPAN E 1000 par SYMRISE,

DEA Methoxycinnamate,

Diisopropyl Methylcinnamate,

Glyceryl Ethylhexanoate Dimethoxycinnamate

### Dérivés de l'acide para-aminobenzoique :

PABA,

Ethyl PABA,

Ethyl Dihydroxypropyl PABA,

Ethylhexyl Diméthyl PABA vendu notamment sous le nom « ESCALOL 507 » par ISP,

Glyceryl PABA,

PEG-25 PABA vendu sous le nom « UVINUL P25 » par BASF,

### Dérivés salicyliques :

Homosalate vendu sous le nom « Eusolex HMS » par Rona/EM Industries,

Ethylhexyl Salicylate vendu sous le nom « NEO HELIOPAN OS » par SYMRISE,

Dipropyleneglycol Salicylate vendu sous le nom « DIPSAL » par SCHER,

TEA Salicylate, vendu sous le nom « NEO HELIOPAN TS » par SYMRISE,

### Dérivés de β,β-diphénylacrylate :

Octocrylene vendu notamment sous le nom commercial « UVINUL N539 » par BASF,

Etocrylene, vendu notamment sous le nom commercial « UVINUL N35 » par BASF,

### Dérivés de la benzophénone :

Benzophenone-1 vendu sous le nom commercial « UVINUL 400 » par BASF,

Benzophenone-2 vendu sous le nom commercial « UVINUL D50 » par BASF

Benzophenone-3 ou Oxybenzone, vendu sous le nom commercial « UVINUL M40 » par BASF,

Benzophenone-4 vendu sous le nom commercial « UVINUL MS40 » par BASF,

Benzophenone-5

Benzophenone-6 vendu sous le nom commercial « Helisorb 11 » par Norquay

Benzophenone-8 vendu sous le nom commercial « Spectra-Sorb UV-24 » par American Cyanamid

Benzophenone-9 vendu sous le nom commercial« UVINUL DS-49» par BASF, Benzophenone-12

2-(4-diethylamino-2-hydroxybenzoyl)-benzoate de n-hexyle vendu sous le nom commercial « UVINUL A+ » par BASF.

### Dérivés du benzylidène camphre :

3-Benzylidene camphor fabriqué sous le nom « MEXORYL SD» par CHIMEX,

4-Methylbenzylidene camphor vendu sous le nom « EUSOLEX 6300 » par MERCK,

Benzylidene Camphor Sulfonic Acid fabriqué sous le nom « MEXORYL SL» par CHIMEX,

Camphor Benzalkonium Methosulfate fabriqué sous le nom « MEXORYL SO » par CHIMEX,

Terephthalylidene Dicamphor Sulfonic Acid fabriqué sous le nom « MEXORYL SX» par CHIMEX,

Polyacrylamidomethyl Benzylidene Camphor fabriqué sous le nom « MEXORYL SW » par CHIMEX,

### Dérivés du phenyl benzimidazole :

Phenylbenzimidazole Sulfonic Acid vendu notamment sous le nom commercial « EUSOLEX 232 » par MERCK,

Disodium Phenyl Dibenzimidazole Tetra-sulfonate vendu sous le nom commercial « NEO HELIOPAN AP » par SYMRISE,

### Dérivés du phenyl benzotriazole :

Drometrizole Trisiloxane vendu sous le nom « Silatrizole » par RHODIA CHIMIE Methylène bis-Benzotriazolyl Tetramethylbutylphénol, vendu sous forme solide sous le nom commercial « MIXXIM BB/100 » par FAIRMOUNT CHEMICAL ou sous forme micronisée en dispersion aqueuse sous le nom commercial « TINOSORB M » par CIBA SPECIALTY CHEMICALS,

### Dérivés anthraniliques :

Menthyl anthranilate vendu sous le nom commercial commercial « NEO HELIOPAN MA » par Haarmann et REIMER,

### Dérivés d'imidazolines :

Ethylhexyl Dimethoxybenzylidene Dioxoimidazoline Propionate,

### Filtres triazines insolubles

- les filtres triazines symétriques décrits dans le brevet US6,225,467, la demande WO2004/085412 (voir composés 6 et 9) ou le document « Symetrical Triazine Derivatives » IP.COM Journal , IP.COM INC WEST HENRIETTA, NY, US (20 septembre 2004) notamment les 2,4,6-tris-(biphényl)-1,3,5-triazines (en particulier la 2,4,6-tris(biphenyl-4-yl-1,3,5-triazine et la 2,4,6-tris(terphenyl)-1,3,5-triazine qui est repris dans les demandes de brevet WO06/035000, WO06/034982, WO06/034991, WO06/035007, WO2006/034992, WO2006/034985.

### Dérivés du benzalmalonate :

Polyorganosiloxane à fonctions benzalmalonate comme le Polysilicone-15 vendu sous la dénomination commerciale « PARSOL SLX » par DSM Nutritional Products, Inc

### Dérivés de 4,4-diarvlbutadiène :

-1,1-dicarboxy (2,2'-diméthyl-propyl)-4,4-diphénylbutadiène

### Les filtres organiques additionnels préférentiels sont choisis parmi

Butyl Methoxydibenzoylmethane
Ethylhexyl Methoxycinnamate
Ethylhexyl Salicylate,
Homosalate,
Octocrylene,
Phenylbenzimidazole Sulfonic Acid,
Benzophenone-3,
2-(4-diethylamino-2-hydroxybenzoyl)-benzoate de n-hexyle.
Terephthalylidene Dicamphor Sulfonic Acid,
Disodium Phenyl Dibenzimidazole Tetra-sulfonate,
Methylène bis-Benzotriazolyl Tetramethylbutylphénol
la 2,4,6-tris(biphenyl-4-yl-1,3,5-triazine
Drometrizole Trisiloxane
Polysilicone-15
Octyl-5-N,N-diethylamino-2-phenysulfonyl-2,4-pentadienoate
   et leurs mélanges.

Les filtres organiques complémentaires conformes à l'invention représentent en général de 0,1 à 30 %, de préférence de 1 à 25 %, du poids total de la composition.

Bien entendu, l'homme de l'art veillera à choisir le ou les éventuels filtres complémentaires et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent également contenir des agents de bronzage et/ou de brunissage artificiels de la peau (agents autobronzants), et plus particulièrement la dihydroxyacétone (DHA). Ils sont présents de préférence dans des quantité allant 0,1 à 10% en poids par rapport au poids total de la composition.

Les compositions aqueuses conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Bien entendu, l'homme de l'art veillera à choisir le ou les éventuels additifs et/ou leurs quantités de manière telle que les propriétés avantageuses attachées intrinsèquement aux compositions conformes à l'invention ne soient pas, ou substantiellement pas, altérées par la ou les adjonctions envisagées.

Les corps gras peuvent être constitués par une huile ou une cire autre que les cires apolaires telles que définies précédemment ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les amides grasses (comme l'isopropyl lauroyl sarcosinate vendu sous la dénomination d' « Eldew SL-205 » par la société Ajinomoto), les acides ou les esters gras comme le benzoate d'alcools en C12-C15 vendu sous la dénomination commerciale « Finsolv TN » ou « Witconol TN » par la société WITCO, le Benzoate de 2-éthylphenyle comme le produit commercial vendu sous le nom X-TEND 226 ® par la société ISP, le palmitate d'octyle, le lanolate d'isopropyle, les triglycérides dont ceux des acides caprique/caprylique, le dicaprylyl carbonate vendu sous la dénomination « Cetiol CC » par la société Cognis), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la cire de carnauba, la cire d'abeille, l'huile de ricin hydrogénée, les cires de polyéthylène et les cires de polyméthylène comme celle vendue sous la dénomination Cirebelle 303 par la société SASOL.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Comme épaississants hydrophiles, on peut citer les polymères carboxyvinyliques tels que les Carbopols (Carbomers) et les Pemulen (Copolymère acrylate/C10-C30-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer / isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination commerciale « Hostacerin AMPS » (nom CTFA : ammonium polyacryloyldimethyl taurate ou le SIMULGEL 800 commercialisé par la société SEPPIC (nom CTFA : sodium polyacryolyldimethyl taurate / polysorbate 80 / sorbitan oleate) ; les copolymères d'acide 2-acrylamido 2-méthylpropane sulfonique et d'hydroxyethyl acrylate comme le SIMULGEL NS et le SEPINOV EMT 10 commercialisés par la société SEPPIC ; les dérivés cellulosiques tels que l'hydroxyéthylcellulose , les polysaccharides et notamment les gommes telles que la gomme de Xanthane ; et leurs mélanges.

Comme épaississants lipophiles, on peut citer les polymères synthétiques tels que les poly C10-C30 alkyl acrylates vendu sous la dénomination « INTELIMER IPA 13-1 » et « INTELIMER IPA 13-6 » par la société Landec ou encore les argiles modifiées telles que l'hectorite et ses dérivés, comme les produits commercialisés sous les noms de Bentone.

Parmi les actifs, on peut citer :
- les vitamines (C, K, PP...) et leurs dérivés ou précurseurs, seuls ou en mélanges,
- les agents anti-pollution et/ou agent anti-radicalaire ;
- les agents dépigmentants et/ou des agents pro-pigmentants ;
- les agents anti-glycation ;
- les agents apaisants,
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants;
- les agents tenseurs,
- les agents matifiants,
- les agents kératolytiques,
- les agents desquamants ;
- les agents hydratants ;
- les agents anti-inflammatoires ;
- les agents agissant sur le métabolisme énergétique des cellules,
- les agents répulsifs contre les insectes
- les antagonistes de substances P ou de CRGP.
- les agents anti-chute et/ou repousse des cheveux
- les agents anti-rides.

Les compositions selon l'invention peuvent être préparées selon les techniques bien connues de l'homme de l'art. Elles peuvent se présenter en particulier sous forme d'émulsion, simple ou complexe (H/E, E/H, H/E/H ou E/H/E) telle qu'une crème, un lait ou d'un gel crème ; sous la forme d'un gel aqueux ; sous la forme d'une lotion. Elles peuvent éventuellement être conditionnées en aérosol et se présenter sous forme de mousse ou de spray.

De préférence, les compositions selon l'invention se présentent sous la forme d'une émulsion huile-dans-eau ou eau-dans huile.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E). Les émulsions peuvent contenir également d'autres types de stabilisants comme par exemple des charges, des polymères gélifiants ou épaississants.

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les diméthicone copolyols tels que le mélange de cyclométhicone et de diméthicone copolyol, vendu sous la dénomination « DC 5225 C » par la société Dow Corning, et les alkyl-dimethicone copolyols tels que le Laurylmethicone copolyol vendu sous la dénomination "Dow Corning 5200 Formulation Aid" par la société Dow Corning ; le Cetyl dimethicone copolyol tel que le produit vendu sous la dénomination Abil EM 90R par la société Goldschmidt et le mélange de cétyl diméthicone copolyol, d'isostéarate de polyglycérole (4 moles) et de laurate d'hexyle vendu sous la dénomination ABIL WE 09 par la société Goldschmidt. On peut y ajouter aussi un ou plusieurs co-émulsionnants, qui, de manière avantageuse, peuvent être choisis dans le groupe comprenant les esters alkylés de polyol.

Comme esters alkylés de polyol, on peut citer notamment les esters de polyéthylèneglycol comme le PEG-30 Dipolyhydroxystearate tel que le produit commercialisé sous le nom Arlacel P135 par la socité ICI .

Comme esters de glycérol et/ou de sorbitan, on peut citer par exemple l'isostéarate de polyglycérol, tel que le produit commercialisé sous la dénomination Isolan GI 34 par la société Goldschmidt ; l'isostéarate de sorbitan, tel que le produit commercialisé sous la dénomination Arlacel 987 par la société ICI ; l'isostéarate de sorbitan et le glycérol, tel que le produit commercialisé sous la dénomination Arlacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) comme le mélange PEG-100 Stearate/ Glyceryl Stearate commercialisé par exemple par la société ICI sous la dénomination Arlacel 165 ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition auto-émulsionnante, comme décrit par exemple dans le document WO-A-92/06778.

Parmi les autres stabilisants d'émulsion, on utilisera plus particulièrement les polymères d'acide isophtalique ou d'acide sulfoisophtalique, et en particulier les copolymères de phtalate / sulfoisophtalate / glycol par exemple le copolymère de Diéthylèneglycol / Phtalate / Isophtalate / 1,4-cyclohexane-diméthanol (nom INCI : Polyester-5) vendu sous les dénominations "Eastman AQ polymer" (AQ35S, AQ38S, AQ55S, AQ48 Ultra) par la société Eastman Chemical.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin, des produits de protection solaire et des produits de maquillage.

Les compositions cosmétiques selon l'invention peuvent par exemple être utilisées comme produit de maquillage.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517.

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Des exemples concrets, mais nullement limitatifs, illustrant l'invention, vont maintenant être donnés.

### SOLUBILITES COMPAREES DE FILTRES TRIAZINES LIPOPHILES ENTRE DES SOLVANTS DE L'ART ANTERIEUR ET LES SOLVANTS DE L'INVENTION

### Filtres testés :

**Filtre 1 :** dineopentyl 2-[4-({4,6-bis[(4-{3-(neopentyloxy)-2-[(neopentyloxy)carbonyl]-3-oxoprop-1-enyl}phenyl)amino]-1,3,5-triazin-2-yl}amino)benzylidene]malonate 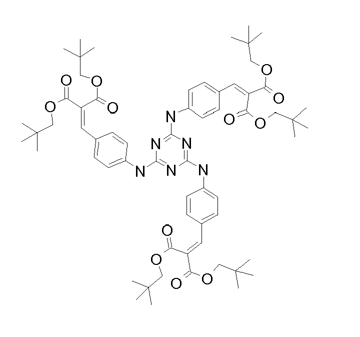
**Filtre 2 :** 2,4-bis-(4'-aminobenzalmalonate d'isobutyle)-6-[(3-{1,3,3,3-tetramethyl-1-[(trimethylsilyl)oxy]disiloxanyl}propyl)amino]-1,3,5-triazine = exemple 5 du brevet EP 0841341 : 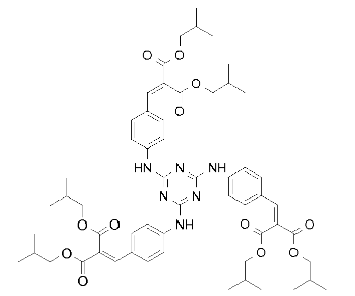
**Filtre 3 :** dineopentyl 2-[4-({4-{[4-(butoxycarbonyl)phenyl]aminol-6-[(4-{3-(neopentyloxy)-2-[(neopentyloxy)carbonyl]-3-oxoprop-1-enyl}phenyl)amino]-1,3,5-triazin-2-yl}amino)benzylidene]malonate 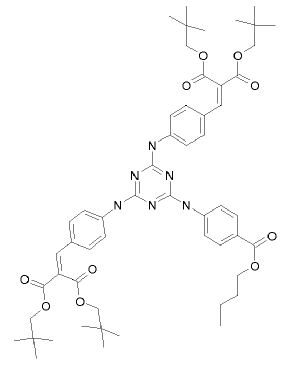
**Filtre 4 :** dineopentyl 2-[4-({4-({4-[(tert-butylamino)carbonyl]phenyl}amino)-6-[(4-{3-(neopentyloxy)-2-[(neopentyloxy)carbonyl]-3-oxoprop-1-enyl}phenyl)amino]-1,3,5-triazin-2-yl}amino)benzylidene]malonate 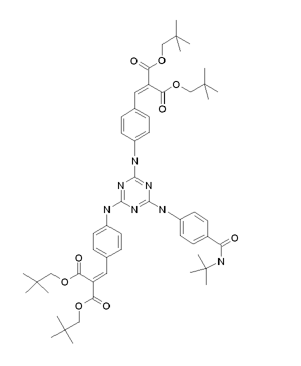
**Filtre 5 :** N-(1,1,3,3-tetramethylbutyl)-4-({4-[(4-{[(1,1,3,3-tetramethylbutyl)amino]carbonyl}phenyl)amino]-6-[(3-{1,3,3,3-tetramethyl-1-[(trimethylsilyl)oxy]disiloxanyl}propyl)amino]-1,3,5-triazin-2-yl}amino)benzamide 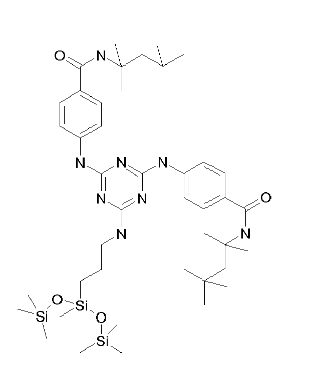
**Filtre 6 :** ethyl 4-({4-{[4-(ethoxycarbonyl)phenyl]amino}-6-[(3-{1,3,3,3-tetramethyl-1-[(trimethylsilyl)oxy]disiloxanyl}propyl)amino]-1,3,5-triazin-2-yl}amino)benzoate 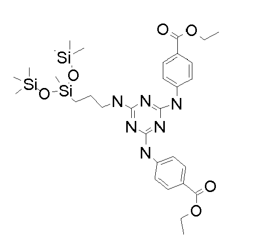
**Filtre 7 :** 2-ethylhexyl 4-({4-({4-[(tert-butylamino)carbonyl]phenyl}amino)-6-[(4-{[(2-ethylhexyl)oxy]carbonyl}phenyl)amino]-1,3,5-triazin-2-yl}amino)benzoate 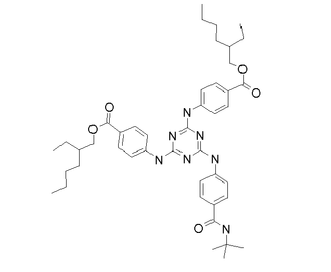
**Filtre 8 :** butyl 4-({4-{[4-(butoxycarbonyl)phenyl]amino}-6-[(3-{1,3,3,3-tetramethyl-1-[(trimethylsilyl)oxy]disiloxanyl}propyl)amino]-1,3,5-triazin-2-yl}amino)benzoate 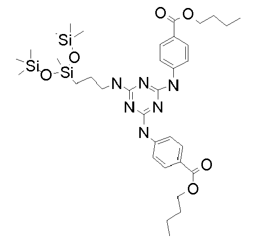
**Filtre 9 :** 2-ethylhexyl 4-({4-({4-[(tert-butylamino)carbonyl]phenyl}amino)-6-[(4-{[(2-ethylhexyl)oxy]carbonyl}phenyl)amino]-1,3,5-triazin-2-yl}amino)benzoate 2-yl}amino)benzoate (connu sous le nom Uvinul T150 de BASF) : 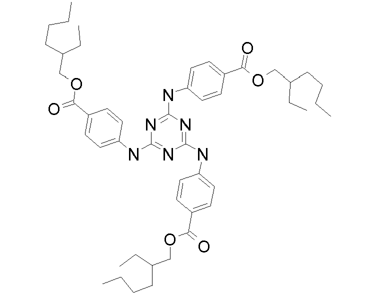
**Filtre 10 :** 5-[(2-ethylhexyl)oxy]-2-[4-{4-[(2-ethylhexyl)oxy]-2-hydroxyphenyl}-6-(4-methoxyphenyl)-1,3,5-triazin-2-yl]phenol (connu sous le nom de Tinosorb S de Ciba) : 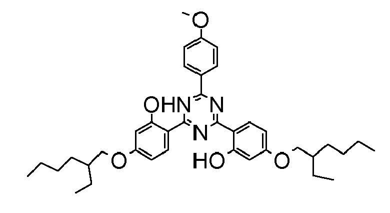
**Huile comparative 1 :** Isopropyl N-lauroylsarcosinate : Eldew SL-205 d' Ajinomoto de formule : 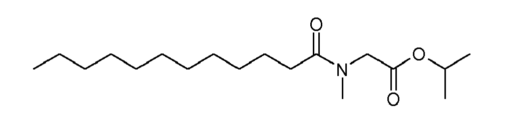
**Huile comparative 2 :** Finsolv TN : Alkylbenzoate en C₁₂-C₁₅
**Huile comparative 3 :** Miglyol 812 : Triglycérides d'acides caprylique-caprique
**Huile comparative 4 :** X-Tend : 2-phenyl ethyl benzoate

### Mode opératoire :

X mg de produit sont introduits dans Y mg d'huile ; avec un léger chauffage (< 60°C) et l'utilisation d'un sonicateur pendant 1 minute, la solution obtenue est laissée à température de labo pendant 1 mois ; l'état de cette solution est observée ; si aucun cristal ou dépôt huileux n'est visible, la solubilité du produit est considérée comme supérieure à Xx100/(X+Y) en poids/poids ; lorsque des cristaux ou un dépôt huileux apparaissent, l'essai est répété avec 5% en moins de produit.

**Tableau 1**

| **Filtre triazine** | **Isopropyl N-lauroyl sarcosinate** | **Composé (m) de l'invention** |
|---|---|---|
| **Filtre 1** | 40% | 60% |
| | Gel | Gel |
| **Filtre 3** | 40% | 60% |
| | Gel | Gel |
| **Filtre 4** | 40% | 60% |
| | Gel | Gel |
| **Filtre 5** | 40% | 60% |
| | Gel | Gel |
| **Filtre 7** | 40% | 60% |
| | Gel | Gel |

**Tableau 2**

| **Huile testée** | **Filtre 2** |
|---|---|
| Isopropyl N-lauroylsarcosinate | 35% |
| | |
| Alkylbenzoate en C₁₂-C₁₅ | 8% |
| | |
| Triglycérides d'acides caprylique-caprique | 6% |
| Composé (m) de l'invention | 60% Gel |

**Tableau 3**

| **Huile testée** | **Filtre 6** |
|---|---|
| Isopropyl N-lauroylsarcosinate | 40% Gel |
| Alkylbenzoate en C₁₂-C₁₅ | 40% Gel |
| | |
| Triglycérides d'acides caprylique-caprique | 40% Gel |
| 2-phenyl ethyl benzoate | 40% Gel |
| Composé (m) de l'invention | 60% Gel |

**Tableau 4**

| **Huile testée** | **Filtre 7** |
|---|---|
| Alkylbenzoate en C₁₂-C₁₅ | 40% Gel |
| | |
| Composé (m) de l'invention | 60% Gel |

**Tableau 5**

| **Huile testée** | **Filtre 8** |
|---|---|
| Isopropyl N-lauroylsarcosinate | 40% Gel |
| Alkylbenzoate en C₁₂-C₁₅ | 28% |
| | |
| Triglycérides d'acides caprylique-caprique | 31% |
| 2-phenyl ethyl benzoate | 27% |
| Composé (m) de l'invention | 60% Gel |
| Composé (j) de l'invention | 60% Gel |
| Composé (I) de l'invention | 60% Gel |
| Composé (ii) de l'invention | 60% Gel |

**Tableau 6**

| **Huile testée** | **Filtre 9** |
|---|---|
| Isopropyl N-lauroylsarcosinate | 40% |
| | |
| Alkylbenzoate en C₁₂-C₁₅ | 5% |
| | |
| Composé (m) de l'invention | 60% Gel |

**Tableau 7**

| **Huile testée** | **Filtre 10** |
|---|---|
| Isopropyl N-lauroyl sarcosinate | 10% |
| Composé (m) de l'invention | 18% |
| Composé (v) de l'invention | 20% |

### EXEMPLES DE FORMULATION 1-4

Les formulations suivantes ont été préparées, les quantités sont exprimées en pourcentages en poids par rapport au poids total de la composition.

| **Composition** | **Ex1** | **Ex2** | **Ex3** | **Ex4** |
|---|---|---|---|---|
| Phase A | | | | |
| Poly Dimethylsiloxane | 0,5 | 0,5 | 0,5 | 0,5 |
| ConservateursConservateurs | 1 | 1 | 1 | 1 |
| Acide Stéarique | 1,5 | 1,5 | 1,5 | 1,5 |
| Mélange mono-stéarate glycéryle / stéarate de PEG100 | 1 | 1 | 1 | 1 |
| Mélange de cétylstéaryl glucoside et d'alcools cétylique, stéarylique | 2 | 2 | 2 | 2 |
| Alcool cétylique | 0,5 | 0,5 | 0,5 | 0,5 |
| Butyl 1-butyl-5-oxopyrrolidine-3-carboxylate (composé (m)) | 20 | | 15 | - |
| Methyl 1-éthyl-5-oxopyrrolidine-3-carboxylate (composé (v)) | | 20 | | 15 |
| bis {ethyl-hexyloxy-2-hydroxy-phenyl}-6-(methoxy-phenyl)-1,3,5-triazine (TINOSORB S -CIBA) | 5 | 5 | | |
| 2,4,6-tris[éthylhexyl-oxycarbonyl)anilino]-1,3,5-triazine (UVINUL T150- BASF) | - | - | 5 | 5 |

| Phase B | | | | |
|---|---|---|---|---|
| Eau désionisée Eau désionisée | QSP 100 | QSP 100 | QSP 100 | QSP 100 |
| Glycérol | 5 | 5 | 5 | 5 |
| Gomme de xanthane | 0,2 | 0,2 | 0,2 | 0,2 |
| Phosphate de mono cétyle | 1 | 1 | 1 | 1 |

| Phase C | | | | |
|---|---|---|---|---|
| Isohexadecane | 1 | 1 | 1 | 1 |
| Copolymère acide acrylique/méthacrylate de stéaryle | 0,2 | 0,2 | 0,2 | 0,2 |
| Triethanolamine | QSP pH | QSP pH | QSP pH | QSP pH |

### Mode opératoire :

On chauffe la phase aqueuse (Phase B) contenant l'ensemble de ses ingrédients à 80°C au bain marie. On chauffe la phase grasse (Phase A) contenant l'ensemble de ses ingrédients à 80°C au bain marie. On émulsionne A dans B sous agitation de type rotor-stator (appareil de la société Moritz). On incorpore la Phase C et on laisse revenir à température ambiante sous agitation modérée. On introduit la triéthanolamine de façon à ajuster le pH à la valeur désirée en fin de fabrication.

Les émulsions antisolaires obtenues sont stables au stockage et ne présentent pas de cristaux ou de précipités.

## Revendications

1. Composition comprenant dans un milieu cosmétiquement acceptable au moins un dérivé ester de 2-pyrrolidinone 4-carboxy de formule (I) suivante : 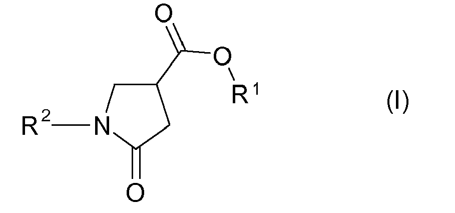 dans laquelle :
R¹ désigne un radical alkyle en C₁-C₂₀, linéaire ou ramifié.
R² désigne un radical alkyle en C₁-C₂₀, linéaire ou ramifié pouvant contenir un cycle en C₅-C₆, le radical phényle, le radical benzyle ou le radical phénéthyle.
et au moins un filtre UV lipophile du type triazine.

2. Composition selon la revendication 1, où le dérivé ester de 2-pyrrolidinone 4-carboxy de formule (I) est choisi parmi les composés suivants :

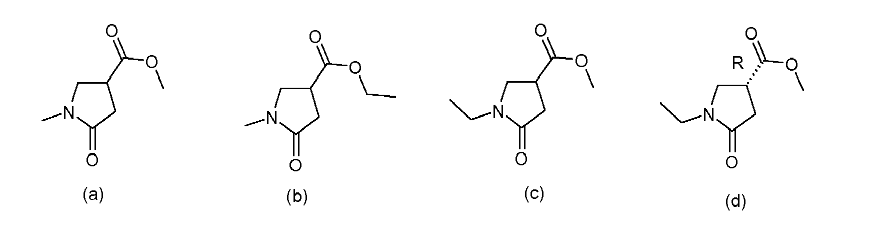

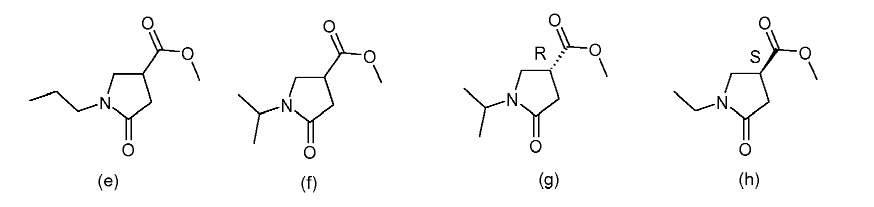

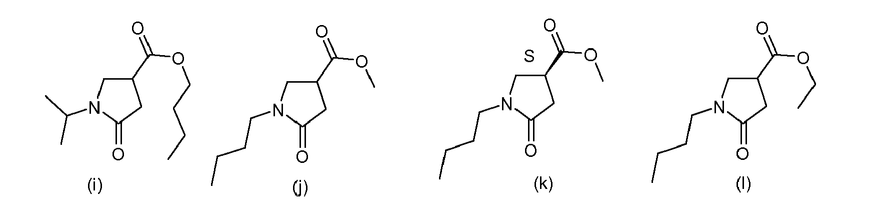

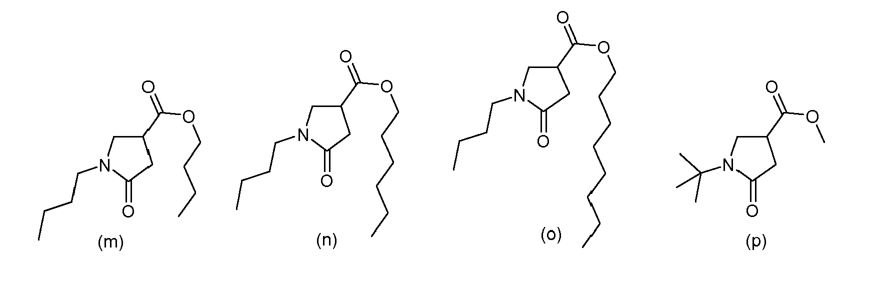

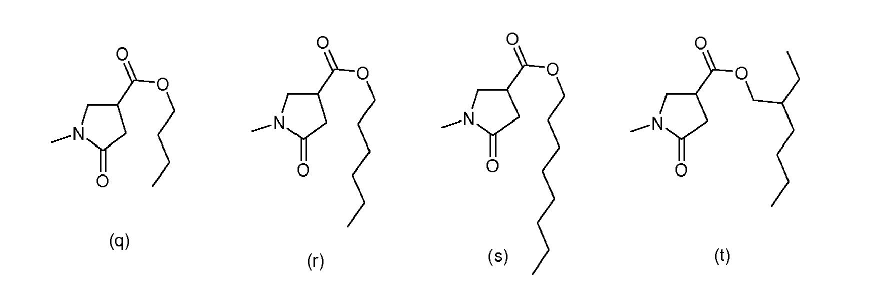

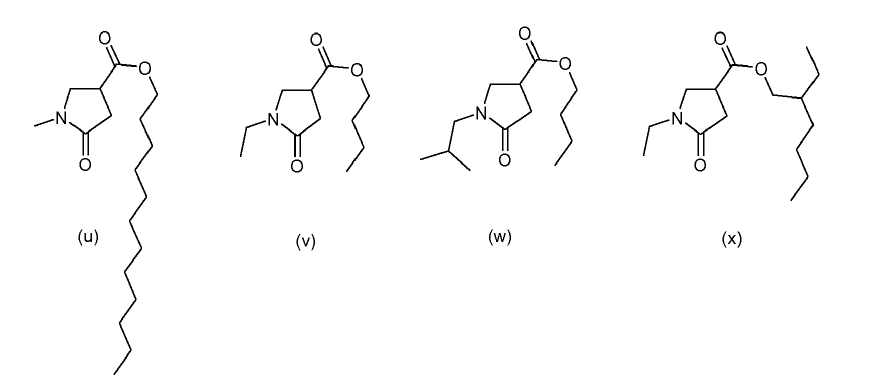

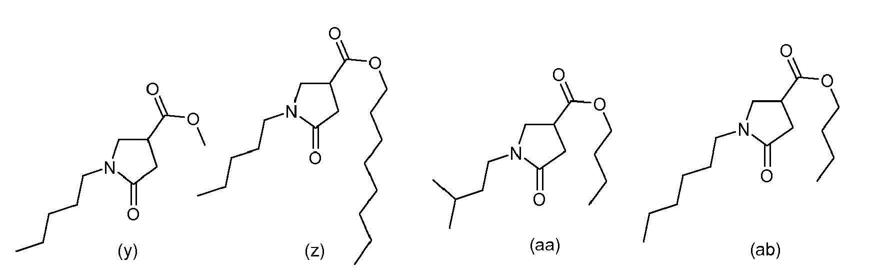

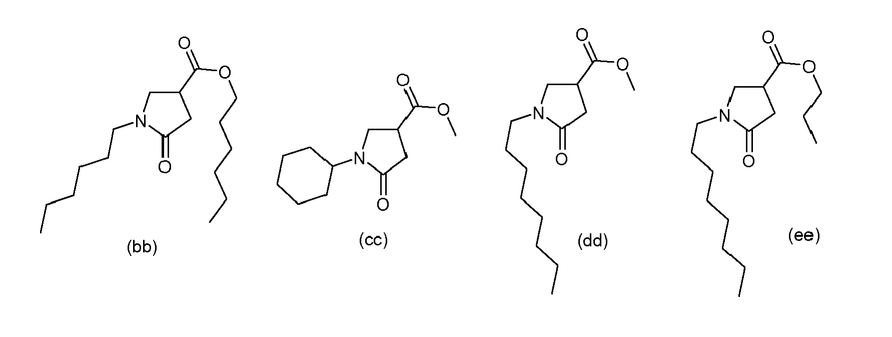

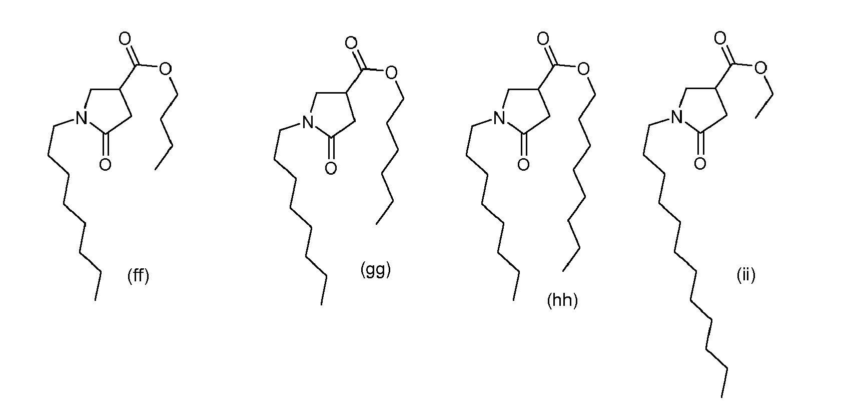

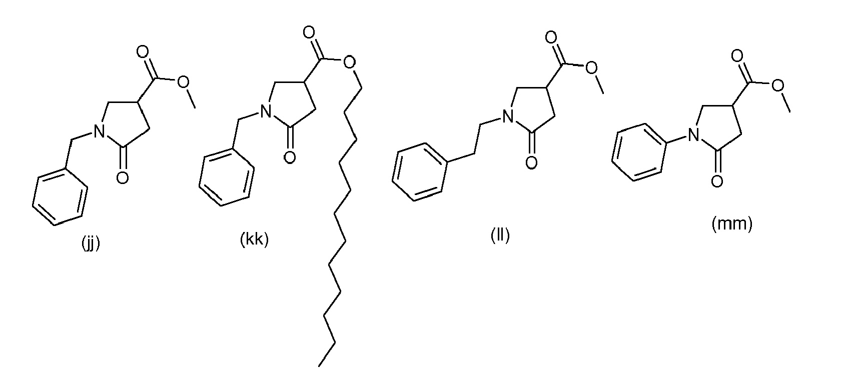

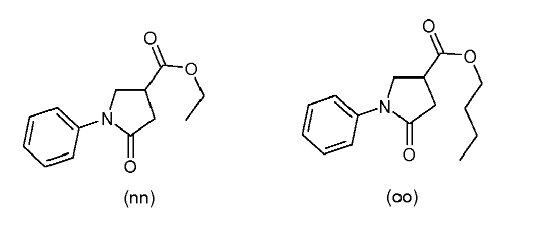

3. Composition selon la revendication 2, où les composés de formule (I) sont choisis parmi les composés (t), (u), (v), (x), (j), (l), (m), (w), (n), (ab), (ii) et (kk) et plus particulièrement parmi les composés (x), (j), (l), (m) et (ii).

4. Composition selon l'une quelconque des revendications 1 à 3, où le filtre UV du type triazine est choisi parmi les composés de formule (1) suivante : 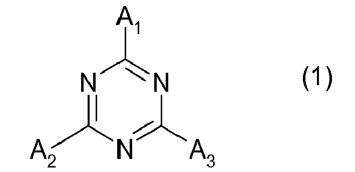 dans laquelle les radicaux A₁, A₂ et A₃, identiques ou différents sont choisis parmi les groupes de formules (2) : 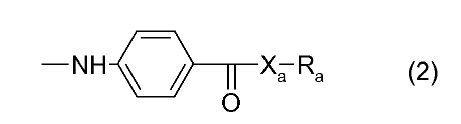 dans laquelle :
- Xₐ, identiques ou différents, représentent l'oxygène ou le radical -NH-;
- Rₐ, identiques ou différents, sont choisis parmi : l'hydrogène; un métal alcalin;
un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé ; un radical de formule (3), (4) ou (5) suivantes : 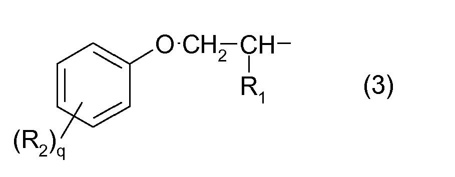 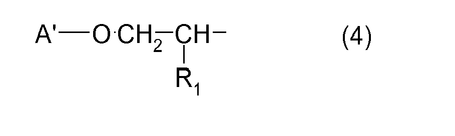 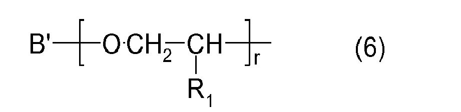 dans lesquelles :
- R₁ est l'hydrogène ou un radical méthyle;
- R₂ est un radical alkyle en C₁-C₉;
- q est un nombre entier allant de 0 à 3;
- r est un nombre entier allant de 1 à 10;
- A' est un radical alkyle en C₄-C₈ ou un radical cycloalkyle en C₅-C₈;
- B' est choisi parmi : un radical alkyle linéaire ou ramifié en C₁-C₈; un radical cycloalkyle en C₅-C₈, un radical aryle éventuellement substitué par un ou plusieurs radicaux alkyles en C₁-C₄.

5. Composition selon la revendication 4, où le filtre triazine de formule (1) est choisi parmi :
- la 2-[(p-(tertiobutylamido)anilino]-4,6-bis-[(p-(2'-éthylhexyl-1'-oxycarbonyl)anilino]-1,3,5-triazine répondant à la formule suivante : 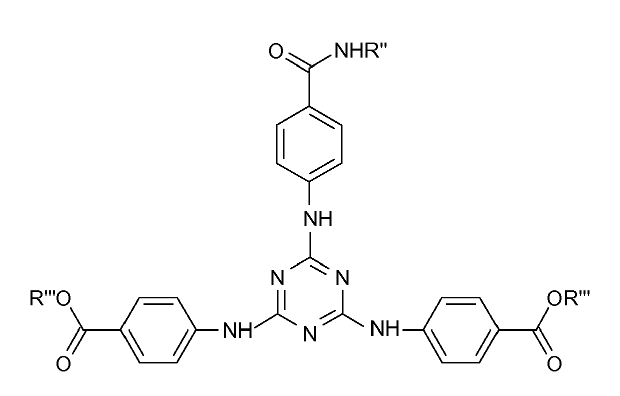
- la 2,4,6-tris[p-(2'-éthylhexyl-1'-oxycarbonyl)anilino]-1,3,5-triazine de formule suivante : 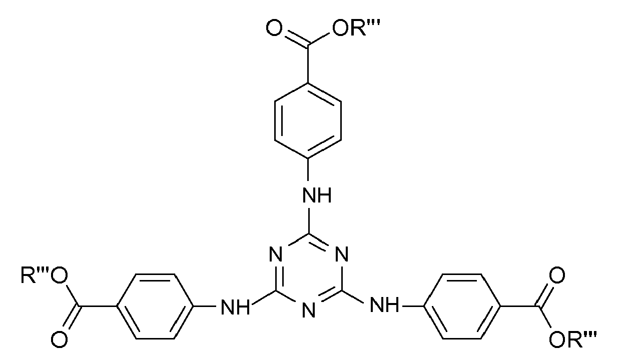 dans laquelle R"' désigne un radical 2-éthyl hexyle.

6. Composition selon l'une quelconque des revendications 1 à 3, où le filtre triazine est choisi parmi les composés de formule (6) suivante : 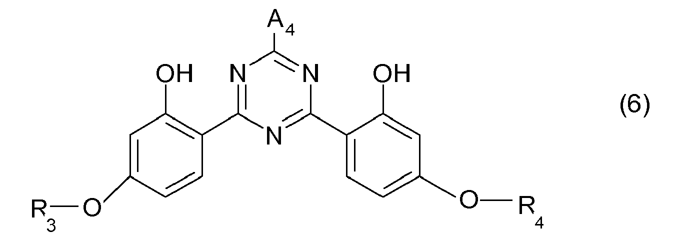 dans laquelle :
les radicaux R₃ et R₄, identiques ou différents, désignent un radical alkyle en C₃-C₁₈; un radical alcényle en C₂-C₁₈ ou bien un reste de formule
-CH₂-CH(OH)-CH₂-OT₁
où T₁ est un atome d'hydrogène ou un radical alkyle en C₁-C₈ ;
- A₄ désigne un reste répondant à l'une des formules suivantes : 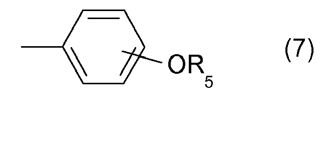 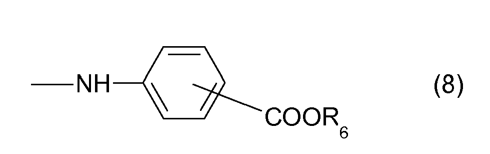 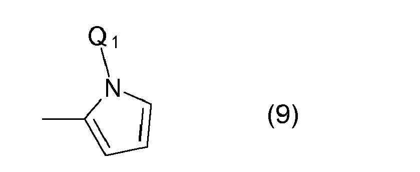 dans lesquelles :
- R₅ désigne un atome d'hydrogène, un radical alkyle en C1-C10, un radical de formule :
-(CH₂CHR₇-O)ₙ₁R₆ où n₁ est un nombre de 1 à 16 ou bien un reste de structure -CH₂-CH-(OH)-CH₂OT₁ avec T₁ ayant la même signification indiquée ci-dessus ;
- R₆ désigne hydrogène, un cation métallique M, un radical alkyle en C₁-C₅ ou un reste de formule -(CH₂)ₘ₂-OT₁ où m₂ est un nombre de 1 à 4 et T₁ a la même signification indiquée ci-dessus.
- R₇ est hydrogène ou méthyle
- Q₁ est un radical alkyle en C₁-C₁₈.

7. Composition selon la revendication 6 où la triazine de formule (6) est la 2,4-bis {[4-(2-éthyl-hexyloxy)-2-hydroxy]-phényl}-6-(4-méthoxy-phényl)-1,3,5-triazine.

8. Composition selon l'une quelconque des revendications 1 à 3, où le filtre triazine est choisi parmi :
2,4,6-tris-(4'-amino benzalmalonate de diisobutyle)-6-butoxy s-triazine,
2,4,6 tris-(4'-amino benzalmalonate de diisobutyle)-6-(2-ethylhexylamino)-s-triazine
2,4-bis-(4'-aminobenzylidène camphre)-6-(2-ethylhexyl-amino)-triazine,
2,4,6 -bis-(4'aminobenzylidène camphre)-6-(4'-aminobenzalmalonate de diisobutyle)-s-triazine,
2,4 6-tris(4'-amino benzalmalonate de diéthyle)-s-triazine
2,4,6-tris-(4'-amino benzalmalonate de diisopropyl)-s-triazine,
2,4,6-tris-(4'-amino benzalmalonate de diisobutyle)-s-triazine
2,4,6-tris(4'-amino benzalmalonate de dinéopentyle)-s-triazine
2,4bis (4'-aminobenzoate de n-butyle)-6-(aminopropyltrisiloxane)-s-triazine,

9. Composition selon l'une quelconque des revendications 1 à 3, où le filtre triazine est choisi parmi les composés de formule générale (9) suivante ou l'une de ses formes tautomères 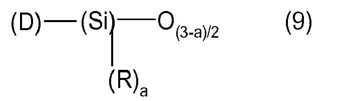 dans laquelle
- R, identiques ou différents représentent un radical alkyle en C₁-C₃₀, linéaire ou ramifié et éventuellement halogéné ou insaturé, un radical aryle en C₆-C₁₂, un radical alkoxy en C₁-C₁₀, un radical hydroxy ou le groupe triméthylsilyloxy ;
- a = 1 à 3 ; en plus des unités de formule -A-(Si)(R)ₐ(O)_{(3-a)/2,}
l'organosiloxane pouvant comporter des unités de formule : (R)_{b}-(Si)(O)_{(4-b)/2} avec b = 1, 2 ou 3 ;
- le groupe (D) désigne un composé s-triazine de formule (10) suivante : 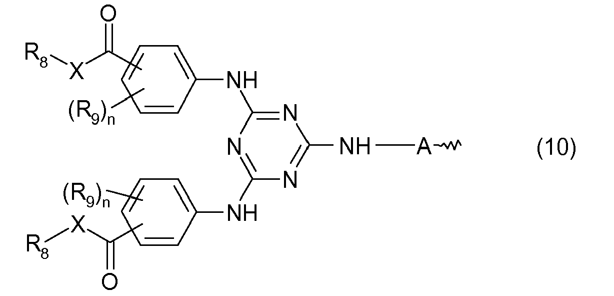 où
- X représente -O- ou -NR₁₀-, avec R₁₀ qui représente l'hydrogène ou un radical alkyle en C₁-C₅,
- R₈ représente un radical alkyle en C₁-C₃₀, linéaire ou ramifié et éventuellement insaturé et pouvant contenir un atome de silicium, un groupe cycloalkyle en C₅-C₂₀, éventuellement substitué par 1 à 3 radicaux alkyles en C₁-C₄, linéaires ou ramifiés, le groupe -(CH₂CHR₁₀-O)ₘR₁₁ ou le groupe -CH₂-CH(OH)-CH₂-O-R₁₂,
- R₉, identiques ou différents, représentent un radical hydroxy, un radical alkyle en C₁-C₈, linéaire ou ramifié, un radical alcoxy en C₁-C₈, deux R₂ adjacents d'un même noyau aromatique pouvant former ensemble un groupement alkylidène dioxy dans lequel le groupe alkylidène contient 1 ou 2 atomes de carbone,
- R₁₀ représente l'hydrogène ou méthyle ; le groupement (C=O)XR₈ pouvant être en position ortho, méta ou para du groupement amino,
- R₁₁ représente l'hydrogène ou un groupe alkyle en C₁-C₈,
- R₁₂ représentent l'hydrogène ou un groupe alkyle en C₄-C₈,
- m est un nombre entier allant de 2 à 20,
- n = 0 à 2,
- A est un radical divalent choisi parmi méthylène ou un groupe répondant à l'une des formules (11), (12) , (13) ou (14) suivantes : 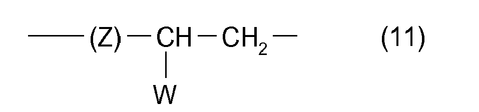 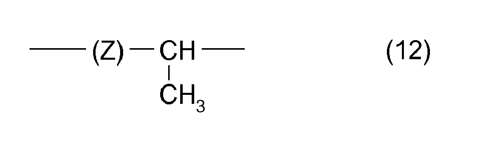 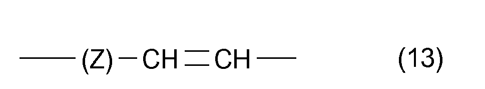 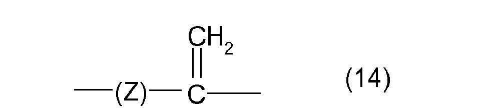 dans lesquelles :
- Z est un diradical alkylène en C₁-C₁₀, linéaire ou ramifié, saturé ou insaturé, éventuellement substitué par un radical hydroxyle ou des oxygènes et pouvant éventuellement contenir un groupement amino,
- W représente un atome d'hydrogène, un radical hydroxyle ou un radical alkyle en C₁-C₈, linéaire ou ramifié, saturé ou insaturé,

10. Composition selon la revendication 9, où la triazine de formule (9) est la 2,4-bis(4'-diylamino benzoate de n-butyle)-6-{[1,3,3,3-tétraméthyl-1 - [(triméthylsilyl)oxy]disiloxanyl]propyl-3-ylamino}-s-triazine de structure (9₂) :

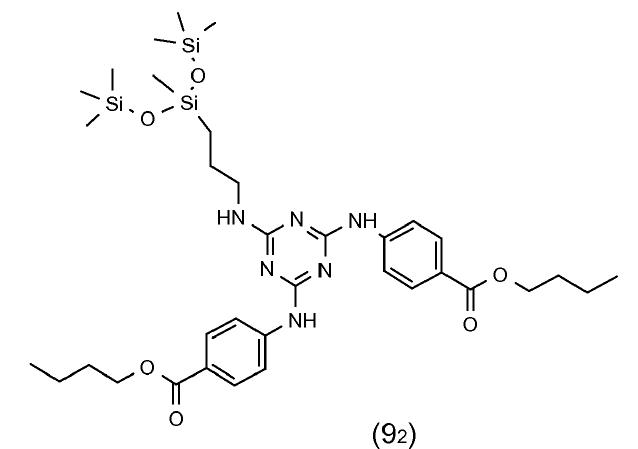

11. Composition selon l'une quelconque des revendications 1 à 3, où le filtre triazine est choisi parmi les composés de formule générale (15) suivante : 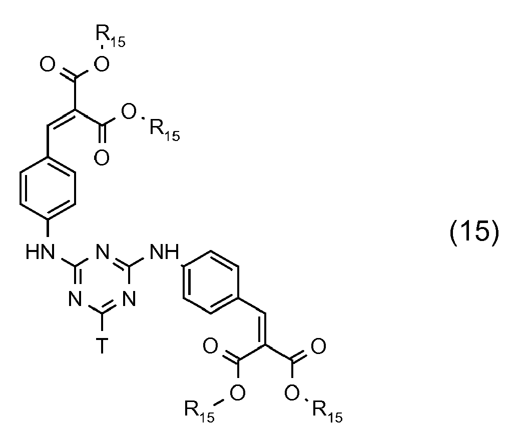 dans laquelle :
R₁₅, identiques ou différents, désignent un groupe de formule (16) : 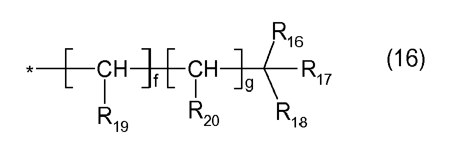 dans laquelle :
R₁₆ et R₁₇, identiques ou différents représentent un groupe alkyle en C₁-C₈, linéaire ou ramifié,
R₁₆ et R₁₇ peuvent former un cycle en C₅-C₈, éventuellement substitué par 1, 2 ou 3 groupements alkyle(s) en C₁-C₄, linéaire(s) ou ramifié(s) ;
R₁₈, R₁₉ et R₂₀, identiques ou différents représentent un atome d'hydrogène ou
un groupe alkyle en C₁-C₄, linéaire ou ramifié ;
f vaut 0 ou 1 ;
g vaut 0 ou 1 ;
sous réserve que :
- lorsque f = 1 et R₁₉ désigne l'hydrogène alors g est égal à 0 et R₁₈ est différent de l'hydrogène,
- lorsque R₁₆ et R₁₇ forment un cycle en C₅-C₈ alors la somme f+g est différente de 2;
T est un radical divalent :
- de formule (17) suivante : 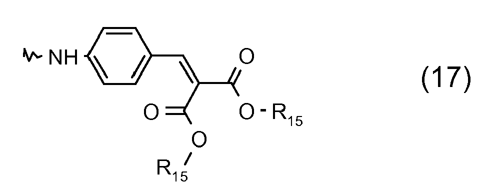 dans laquelle R₁₅ a la même définition que dans la formule (15) ci-dessus ;
- de formule (18) suivante : 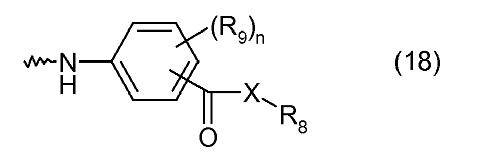 dans laquelle R₈, R₉, X et n ont la même définition que dans la formule (9) dans la revendication 9 ;
- de formule (19) suivante : 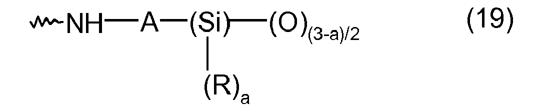
- ou de formule (20) suivante : 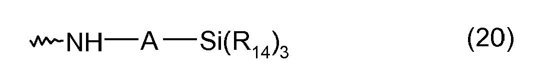 dans laquelle A et R₁₄ ont les mêmes définitions que dans la formule (9c) dans la revendication 9.

12. Composition selon la revendication 11, où la triazine de formule (15) est choisi parmi :
- le dineopentyl 2-[4-({4,6-bis[(4-{3-(neopentyloxy)-2-[(neopentyloxy)carbonyl]-3-oxoprop-1-enyl}phenyl)amino]-1,3,5-triazin-2-yl}amino)benzylidene]malonate de formule (15₁) : 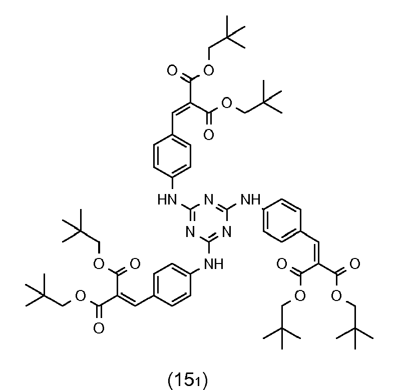
- la 2,4-bis-(4'-amino benzalmalonate de di-néopentyle)-6-(4"-amino benzoate de butyle)-s-triazine de formule (15₆).

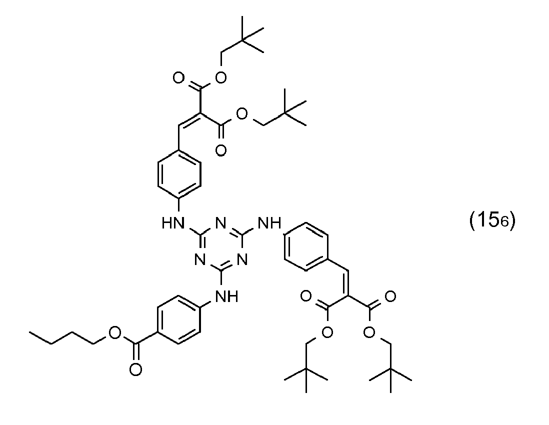

13. Composition selon l'une quelconque des revendications 1 à 12, où le ou les dérivés de formule (I) sont présents dans une quantité suffisante permettant de solubiliser à lui-seul ou à eux-seuls la quantité totale de filtre(s) triazine(s) lipophile(s) présent(s) dans la composition.

14. Utilisation d'au moins un dérivé ester de 2-pyrrolidinone 4-carboxy de formule (I) tel que défini dans l'une quelconque des revendications précédentes, dans une composition comprenant dans un milieu cosmétiquement acceptable au moins un filtre UV lipophile du type triazine tel que défini dans l'une quelconque des revendications précédentes, comme solvant dudit filtre UV triazine dans et/ou comme agent améliorant la solubilité dudit filtre UV triazine dans ladite composition.

15. Utilisation d'au moins un dérivé diester de pyrrolidinone de formule (I) tel que défini dans l'une quelconque des revendications précédentes dans une composition comprenant dans un milieu cosmétiquement acceptable au moins un filtre UV lipophile du type triazine tel que défini dans l'une quelconque des revendications précédentes dans le but d'améliorer le facteur de protection solaire.
